# FASCICULE DE BREVET EUROPEEN

(11) **EP 1 877 409 B1**
(45) Date de publication et mention de la délivrance du brevet: **17.06.2009**
(21) Numéro de dépôt: 06743743.4
(22) Date de dépôt: 26.04.2006
(51) Int. Cl.: C07D 471/04, A61K 31/437, A61P 35/00

(54) **PYRROLOPYRIDINES SUBSTITUES, COMPOSITIONS LES CONTENANT, PROCEDE DE FABRICATION ET UTILISATION**
SUBSTITUIERTE PYRROLOPYRIDINE, ZUSAMMENSETZUNGEN DAMIT UND VERFAHREN ZU IHRER HERSTELLUNG UND VERWENDUNG
SUBSTITUTED PYRROLO-PYRIDINES, COMPOSITION CONTAINING THEM, METHOD FOR THEIR PRODUCING AND USE THEREOF

(30) Priorité: 26.04.2005 FR 0504173
(43) Date de publication de la demande: 16.01.2008
(73) Titulaire: Aventis Pharma S.A., 92160 Antony (FR)
(72) Inventeur: TABART, Michel, F-91290 La Norville (FR); BACQUE, Eric, F-91190 Gif Sur Yvette (FR); HALLEY, Frank, F-92370 Chaville (FR); RONAN, Baptiste, F-92140 Clamart (FR); DESMAZEAU, Pascal, F-91250 Tigery (FR); VIVIANI, Fabrice, F-95380 Louvres (FR); SOUAILLE, Catherine, F-94600 Choisy Le Roi (FR)
(74) Mandataire: Le Pennec, Magali
(86) Numéro de dépôt international: PCT/FR2006/000925
(87) Numéro de publication internationale: WO 2006/114520

(56) Documents cités:
- WO-A-01/98299
- WO-A-03/000688
- WO-A-03/028724
- WO-A-03/082868

## Description

La présente invention concerne notamment de nouveaux composés chimiques, particulièrement des pyrrolopyridines substituées, les compositions les contenant, et leur utilisation comme médicaments.

Plus particulièrement, et selon un premier aspect, l'invention concerne de nouvelles pyrrolopyridines substituées spécifiques présentant une activité anticancéreuse, via la modulation de l'activité de protéines, en particulier des kinases.

A ce jour, la plupart des composés commerciaux utilisés en chimiothérapie posent des problèmes importants d'effets secondaires et de tolérance par les patients. Ces effets pourraient être limités dans la mesure où les médicaments utilisés agissent sélectivement sur les cellules cancéreuses, à l'exclusion des cellules saines. Une des solutions pour limiter les effets indésirables d'une chimiothérapie peut donc consister en l'utilisation de médicaments agissant sur des voies métaboliques ou des éléments constitutifs de ces voies, exprimés majoritairement dans les cellules cancéreuses, et qui ne seraient pas ou peu exprimés dans les cellules saines.

Les protéines kinases sont une famille d'enzymes qui catalysent la phosphorylation de groupes hydroxyles de résidus spécifiques de protéines tels que des résidus tyrosine, sérine ou thréonine. De telles phosphorylations peuvent largement modifier la fonction des protéines ; ainsi, les protéines kinases jouent un rôle important dans la régulation d'une grande variété de processus cellulaires, incluant notamment le métabolisme, la prolifération cellulaire, la différentiation cellulaire, la migration cellulaire ou la survie cellulaire. Parmi les différentes fonctions cellulaires dans lesquelles l'activité d'une protéine kinase est impliquée, certains processus représentent des cibles attractives pour traiter les maladies cancéreuses ainsi que d'autres maladies.

Ainsi, un des objets de la présente invention est de proposer des compositions ayant une activité anticancéreuse, en agissant en particulier vis-à-vis de kinases. Parmi les kinases pour lesquelles une modulation de l'activité est recherchée, KDR et Tie2 sont préférées.

Ces produits répondent à la formule (1) suivante : 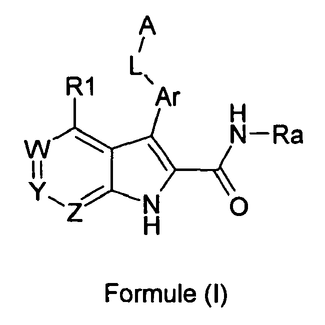 dans laquelle :
1) A et Ar sont des groupes phényle substitués;
2) L est NH-CO-NH;
3) W est C(R6), l'un de Y et Z, est choisi parmi N et NO, et l'autre est C(R5) et ;
4) R1, R5, et R6 sont H ;
5) Ra est H.

Des combinaisons acceptables de substituants incluent celles dans lesquelles R1, R5 et R6 sont H et l'un de Y et Z est choisi parmi N et NO.

A est avantageusement substitué par un premier substituant sélectionné dans le groupe constitué par (C1-C12)alkyle, (C1-C12)alkyle halogéné, (C3-C12)cycloalkyle, (C2-C12)alkylène, (C2-C12)alkynyle, (C8-C14)aryle, (C1-C13)hétéroaryle, O-(C1-C3)alkyle, O-(C6-C14)Aryle, O-(C1-C13)hétéroaryle, S-(C1-C3)alkyle, S-(C6-C14)Aryle, S-(C1-C13)hétéroaryle, chacun étant éventuellement substitué par un substituant choisi parmi (C1-C3)alkyle, halogène, O-(C1-C3)alkyle, N(R8)(R9) ; dans lequel R8 et R9 sont indépendamment choisis parmi H, (C1-C3)alkyle, (C1-C3)alkyleOH, (C1-C3)alkyle halogéné, (C1-C3)alkyleNH₂, (C1-C3)alkyleCOOM, (C1-C3)alkyleSO₃M , dans lequel lorsque R8 et R9 sont simultanément différents de H, ils peuvent être liés pour former un cycle de 5 à 7 chaînons comportant de 0 à 3 hétéroatomes choisis parmi O, N et S, dans lequel M est H ou un cation de métal alcalin choisi parmi Li, Na et K.

En outre, A est aussi avantageusement substitué par un deuxième substituant sélectionné dans le groupe constitué par F, Cl, Br, I, OH, SH, SO₃M COOM, CN, NO₂, CON(R8)(R9), N(R8)CO(R9), (C1-C3)alkyle-OH, (C1-C3)alkyle-N(R8)(R9), (C1-C3)alkyle-(R10), (C1-C3)alkyle-COOH, N(R8)(R9) ; dans lequel R8 et R9 sont indépendamment choisis parmi H, (C1-C3)alkyle, (C1-C3)alkyleOH, (C1-C3)alkyle halogéné, (C1-C3)alkyleNH₂, (C1-C3)alkyleCOOM, (C1-C3)alkyleSO₃M ; dans lequel lorsque R8 et R9 sont simultanément différents de H, ils peuvent être liés pour former un cycle de 5 à 7 chaînons comportant de 0 à 3 hétéroatomes choisis parmi O, N et S; dans lequel M est H ou un cation de métal alcalin choisi parmi Li, Na et K ; et dans lequel R10 est H ou un hétérocycle non aromatique éventuellement substitué, comprenant 2 à 7 atomes de carbone, et 1 à 3 hétéroatomes choisis parmi N, O et S.

Lorsque A est disubstitué, les deux substituants peuvent être liés entre eux pour former un cycle de 5 à 7 chaînons contenant de 0 à 3 hétéroatomes choisis parmi N, O et S.

Selon un mode de réalisation préféré, A est phényle substitué par au moins un groupe choisi parmi halogène, (C1-C4)alkyle, (C1-C3)alkyle halogéné, O-(C1-C4)alkyle, S-(C1-C4)alkyle, O-(C1-C4)alkyle halogéné, S-(C1-C4)alkyle halogéné, et dans laquelle lorsque A est disubstitué, les deux substituants peuvent être liés entre eux pour former un cycle de 5 à 7 chaînons contenant de 0 à 3 hétéroatomes choisis parmi N, O et S.

Les produits selon l'invention peuvent être sous forme :
1) non chirale, ou
2) racémique, ou
3) enrichie en un stéréo-isomère, ou
4) enrichie en un énantiomère ;
(C1-C3)alkyleCOOM, (C1-C3)alkyleSO₃M ; dans lequel lorsque R8 et R9 sont simultanément différents de H, ils peuvent être liés pour former un cycle de 5 à 7 chaînons comportant de 0 à 3 hétéroatomes choisis parmi O, N et S, dans lequel M est H ou un cation de métal alcalin choisi parmi Li, Na et K.

En outre, A est aussi avantageusement substitué par un deuxième substituant sélectionné dans le groupe constitué par F, CI, Br, I, OH, SH, SO₃M, COOM, CN, NO₂, CON(R8)(R9), N(R8)CO(R9), (C1-C3)alkyle-OH, (C1-C3)alkyle-N(R8)(R9), (C1-C3)alkyle-(R10), (C1-C3)alkyle-COOH, N(R8)(R9) ; dans lequel R8 et R9 sont indépendamment choisis parmi H, (C1-C3)alkyle, (C1-C3)alkyleOH, (C1-C3)alkyle halogéné, (C1-C3)alkyleNH₂, (C1-C3)alkyleCOOM, (C1-C3)alkyleSO₃M ; dans lequel lorsque R8 et R9 sont simultanément différents de H, ils peuvent être liés pour former un cycle de 5 à 7 chaînons comportant de 0 à 3 hétéroatomes choisis parmi O, N et S; dans lequel M est H ou un cation de métal alcalin choisi parmi Li, Na et K ; et dans lequel R10 est H ou un hétérocycle non aromatique éventuellement substitué, comprenant 2 à 7 atomes de carbone, et 1 à 3 hétéroatomes choisis parmi N, O et S.

Lorsque A est disubstitué, les deux substituants peuvent être liés entre eux pour former un cycle de 5 à 7 chaînons contenant de 0 à 3 hétéroatomes choisis parmi N, O et S.

Selon un mode de réalisation préféré, A est phényle, pyrazolyle ou isoxazolyle substitué par au moins un groupe choisi parmi halogène, (C1-C4)alkyle, (C1-C3)alkyle halogéné, O-(C1-C4)alkyle, S-(C1-C4)alkyle, O-(C1-C4)alkyle halogéné, S-(C1-C4)alkyle halogéné, et dans laquelle lorsque A est disubstitué, les deux substituants peuvent être liés entre eux pour former un cycle de 5 à 7 chaînons contenant de 0 à 3 hétéroatomes choisis parmi N, O et S.

Les produits selon l'invention peuvent être sous forme :
1) non chirale, ou
2) racémique, ou
3) enrichie en un stéréo-isomère, ou
4) enrichie en un énantiomère ;
et être éventuellement salifiés.

Un produit conforme à l'invention pourra être utilisé pour la fabrication d'un médicament utile pour traiter un état pathologique, en particulier un cancer.

La présente invention concerne aussi un médicament comprenant un produit selon l'invention, et les compositions thérapeutiques comprenant un produit selon l'invention en combinaison avec un excipient pharmaceutiquement acceptable selon le mode d'administration choisi. La composition pharmaceutique peut se présenter sous forme solide, liquide ou de liposomes.

Parmi les compositions solides on peut citer les poudres, les gélules, les comprimés. Parmi les formes orales on peut aussi inclure les formes solides protégées vis-à-vis du milieu acide de l'estomac. Les supports utilisés pour les formes solides sont constitués notamment de supports minéraux comme les phosphates, les carbonates ou de supports organiques comme le lactose, les celluloses, l'amidon ou les polymères. Les formes liquides sont constituées de solutions de suspensions ou de dispersions. Elles contiennent comme support dispersif soit l'eau, soit un solvant organique (éthanol, NMP ou autres) ou de mélanges d'agents tensioactifs et de solvants ou d'agents complexants et de solvants.

Les formes liquides seront, de préférence, injectables et de ce fait auront une formulation acceptable pour une telle utilisation.

Des voies d'administration par injection acceptables incluent les voies intraveineuse, intra-péritonéale, intramusculaire, et sous cutanée, la voie intraveineuse étant habituellement préférée.

La dose administrée des composés de l'invention sera adaptée par le praticien en fonction de la voie d'administration au patient et de l'état de ce dernier.

Les composés de la présente invention peuvent être administrés seuls ou en mélange avec d'autres anticancéreux. Parmi les associations possibles on peut citer:
- les agents alkylants et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmélamine
- les dérivés du platine comme notamment le cisplatine, le carboplatine ou l'oxaliplatine
- les agents antibiotiques comme notamment la bléomycine, la mitomycine, la dactinomycine
- les agents antimicrotubules comme notamment la vinblastine, la vincristine, la vindésine, la vinorelbine, les taxoïdes (paclitaxel et docétaxel)
- les anthracyclines comme notamment la doxorubicine, la daunorubicine, l'idarubicine, l'épirubicine, la mitoxantrone, la losoxantrone
- les inhibiteurs de topoisomérases des groupes I et II telles que l'étoposide, le teniposide, l'amsacrine, l'irinotecan, le topotecan et le tomudex
- les fluoropyrimidines telles que la 5-fluorouracile, l'UFT, la floxuridine
- les analogues de cytidine telles que la 5-azacytidine, la cytarabine, la gemcitabine, la 6-mercaptomurine, la 6-thioguanine
- les analogues d'adénosine tels que la pentostatine, la cytarabine ou le phosphate de fludarabine
- le méthotrexate et l'acide folinique
- les enzymes et composés divers tels que la L-asparaginase, l'hydroxyurée, l'acide trans-rétinoique, la suramine, la

dexrazoxane, l'amifostine, l'herceptine ainsi que les hormones oestrogéniques, androgéniques
- les agents antivasculaires tels que les dérivés de la combretastatine, par exemple la CA4P, des chalcones ou de la colchicine, par exemple le ZD6126, et leurs prodrogues.

Il est également possible d'associer aux composés de la présente invention un traitement par des radiations. Ces traitements peuvent être administrés simultanément, séparément, séquentiellement. Le traitement sera adapté par le praticien en fonction du malade à traiter.

Les produits de l'invention sont utiles comme agents inhibiteurs d'une réaction catalysée par une kinase, en particulier FAK, KDR, Tie2, Aurora A, Aurora B et CDK2. FAK, KDR et Tie2 sont des kinases pour lesquelles les produits de l'invention seront particulièrement utiles en tant qu'inhibiteurs.

Les raisons pour lesquelles ces kinases sont choisies sont données ci-après :

### FAK

FAK est une tyrosine kinase cytoplasmique jouant un rôle important dans la transduction du signal transmis par les intégrines, famille de récepteurs hétérodimériques de l'adhésion cellulaire. FAK et les intégrines sont colocalisés dans des structures périmembranaires appelées plaques d'adhérence. Il a été montré dans de nombreux types cellulaires que l'activation de FAK ainsi que sa phosphorylation sur des résidus tyrosine et en particulier son autophosphorylation sur la tyrosine 397 étaient dépendantes de la liaison des intégrines à leurs ligands extracellulaires et donc induites lors de l'adhésion cellulaire [Kornberg L, et al. J. Biol. Chem. 267(33): 23439-442. (1992)]. L'autophosphorylation sur la tyrosine 397 de FAK représente un site de liaison pour une autre tyrosine kinase, Src, via son domaine SH2 [Schaller et al. Mol. Cell. Biol. 14 :1680-1688. 1994; Xing et al. Mol. Cell. Biol. 5 :413-421. 1994]. Src peut alors phosphoryler FAK sur la tyrosine 925, recrutant ainsi la protéine adaptatrice Grb2 et induisant dans certaines cellules l'activation de la voie ras et MAP Kinase impliquée dans le contrôle de la prolifération cellulaire [Schlaepfer et al. Nature; 372:786-791. 1994; Schlaepfer et al. Prog. Biophy. Mol. Biol. 71:435-478. 1999; Schlaepfer and Hunter, J. Biol. Chem. 272:13189-13195. 1997]. L'activation de FAK peut aussi induire la voie de signalisation jun NH2-terminal kinase (JNK) et résulter dans la progression des cellules vers la phase G1 du cycle cellulaire [Oktay et al., J. Cell. Biol.145 :1461-1469. 1999]. Phosphatidylinositol-3-OH kinase (P13-kinase) se lie aussi à FAK sur la tyrosine 397 et cette interaction pourrait être nécessaire à l'activation de P13-kinase [Chen and Guan, Proc. Nat. Acad. Sci. USA. 91: 10148-10152. 1994; Ling et al. J. Cell. Biochem. 73 :533-544. 1999]. Le complexe FAK/Src phosphoryle différents substrats comme la paxilline et p130CAS dans les fibroblastes [Vuori et al. Mol. Cell. Biol. 16: 2606-2613. 1996].

Les résultats de nombreuses études soutiennent l'hypothèse que les inhibiteurs de FAK pourraient être utiles dans le traitement du cancer. Des études ont suggéré que FAK puisse jouer un rôle important dans la prolifération et/ou la survie cellulaire *in vitro.* Par exemple, dans les cellules CHO, certains auteurs ont démontré que la surexpression de p125FAK conduit à une accélération de la transition G1 à S, suggérant que p125FAK favorise la prolifération cellulaire [Zhao J.-H et al. J. Cell Biol. 143:1997-2008. 1998]. D'autres auteurs ont montré que des cellules tumorales traitées avec des oligonucléotides anti-sens de FAK perdent leur adhésion et entrent en apoptose (Xu et al, Cell Growth Differ. 4:413-4.18. 1996). Il a également été démontré que FAK promeut la migration des cellules *in vitro.* Ainsi, des fibroblastes déficients pour l'expression de FAK (souris « knockout » pour FAK) présentent une morphologie arrondie, des déficiences de migration cellulaire en réponse à des signaux chimiotactiques et ces défauts sont supprimés par une réexpression de FAK [DJ. Sieg et al., J. Cell Science. 112:2677-91. 1999]. La surexpression du domaine C-terminal de FAK (FRNK) bloque l'étirement des cellules adhérentes et réduit la migration cellulaire *in vitro* [Richardson A. and Parsons J.T. Nature. 380:538-540. 1996]. La surexpression de FAK dans des cellules CHO, COS ou dans des cellules d'astrocytome humain favorise la migration des cellules. L'implication de FAK dans la promotion de la prolifération et de la migration des cellules dans de nombreux types cellulaires *in vitro,* suggère le rôle potentiel de FAK dans les processus néoplasiques. Une étude récente a effectivement démontré l'augmentation de la prolifération des cellules tumorales *in vivo* après induction de l'expression de FAK dans des cellules d'astrocytome humain [Cary L.A. et al. J. Cell Sci. 109:1787-94. 1996; Wang D et al. J. Cell Sci. 113:4221-4230. 2000]. De plus, des études immunohistochimiques de biopsies humaines ont démontré que FAK était surexprimé dans les cancers de la prostate, du sein, de la thyroïde, du colon, du mélanome, du cerveau et du poumon, le niveau d'expression de FAK étant directement corrélé aux tumeurs présentant le phénotype le plus agressif [Weiner TM, et al. Lancet. 342(8878):1024-1025. 1993 ; Owens et al. Cancer Research. 55:2752-2755. 1995; Maung K. et al. Oncogene. 18:6824-6828. 1999; Wang D et al. J. Cell Sci. 113:4221-4230. 2000].

### KDR

KDR (Kinase insert Domain Receptor) aussi appelée VEGF-R2 (Vascular Endothelial Growth Factor Receptor 2), est exprimé uniquement dans les cellules endothéliales. Ce récepteur se fixe au facteur de croissance angiogénique VEGF, et sert ainsi de médiateur à un signal transductionnel via l'activation de son domaine kinase intracellulaire. L'inhibition directe de l'activité kinase de VEGF-R2 permet de réduire le phénomène d'angiogénèse en présence de VEGF exogène (Vascular Endothelial Growth Factor : facteur de croissance vasculaire endothélial) (Strawn et al., Cancer Research, 1996, vol. 56, p.3540-3545). Ce processus a été démontré notamment à l'aide de mutants VEGF-R2 (Millauer et al., Cancer Research, 1996, vol. 56, p.1615-1620). Le récepteur VEGF-R2 semble n'avoir aucune autre fonction chez l'adulte que celle liée à l'activité angiogénique du VEGF. Par conséquent, un inhibiteur sélectif de l'activité kinase du VEGF-R2 ne devrait démontrer que peu de toxicité.

En plus de ce rôle central dans le processus dynamique angiogénique, des résultats récents suggèrent que l'expression de VEGF contribue à la survie des cellules tumorales après des chimio- et radio-thérapies, soulignant la synergie potentielle d'inhibiteurs de KDR avec d'autres agents (Lee et al. Cancer Research, 2000, vol. 60, p.5565-5570).

### Tie2

Tie-2 (TEK) est un membre d'une famille de récepteurs à tyrosine kinase, spécifique des cellules endothéliales. Tie2 est le premier récepteur à activité tyrosine kinase dont on connaît à la fois l'agoniste (angiopoïétine 1 ou Ang1) qui stimule l'autophosphorylation du récepteur et la signalisation cellulaire [S. Davis et al (1996) Cell 87, 1161-1169] et l'antagoniste (angiopoïétine 2 ou Ang2) [P.C. Maisonpierre et al. (1997) Science 277, 55-60]. L'angiopoïétine 1. peut avoir un effet synergique avec le VEGF dans les derniers stades de la néo-angiogénèse *[*AsaharaT. Circ. Res.(1998) 233-240]. Les expériences de knock-out et les manipulations transgéniques de l'expression de Tie2 ou de Ang1 conduisent à des animaux qui présentent des défauts de vascularisation [D.J. Dumont et al (1994) Genes Dev. 8, 1897-1909 *et* C. Suri (1996) Cell 87, 1171-1180]*.* La liaison d'Ang1 à son récepteur conduit à l'autophosphorylation du domaine kinase de Tie2 qui est essentielle pour la néovascularisation ainsi que pour le recrutement et l'interaction des vaisseaux avec les péricytes et les cellules musculaires lisses ; ces phénomènes contribuent à la maturation et la stabilité des vaisseaux nouvellement formés [P. C. Maisonpierre et al (1997) Science 277, 55-60]*.* Lin et al (1997) J. Clin. Invest. 100, 8: 2072-2078 *et* Lin P. (1998) PNAS 95, 8829-8834, ont montré une inhibition de la croissance et de la vascularisation tumorale, ainsi qu'une diminution des métastases de poumon, lors d'infections adénovirales ou d'injections du domaine extracellulaire de Tie-2 (Tek) dans des modèles de xénogreffes de tumeur du sein et de mélanome.

Les inhibiteurs de Tie2 peuvent être utilisés dans les situations où une néovascularisation se fait de façon inappropriée (c'est-à-dire dans la rétinopathie diabétique, l'inflammation chronique, le psoriasis, le sarcome de Kaposi, la néovascularisation chronique due à la dégénérescence maculaire, l'arthrite rhumatoïde, l'hémoangiome infantile et les cancers).

La progression du cycle cellulaire est souvent gérée par des kinases cycline dépendantes (**CDK**) qui sont activées par une interaction avec des proteines appartenant à la famille des cyclines, activation qui se termine par la phosphorylation de substrats et finalement par la division cellulaire. En plus les inhibiteurs endogènes des CDK qui sont activés (famille des INK4 et des KIP/CIP) régulent de façon négative l'activité des CDK. La croissance des cellules normales est due à une balance entre les activateurs des CDK (les cyclines) et les inhibiteurs endogènes des CDK. Dans plusieurs types de cancers, l'expression ou l'activité aberrante de plusieurs de ces régulateurs du cycle cellulaire a été décrite.

La cycline E active la kinase Cdk2 qui agit ensuite pour phosphoryler la protéine pRb (protéine du rétinoblastome) résultant en un engagement dans la division cellulaire irréversible et une transition vers la phase S (PL Toogood, Medicinal Research Reviews (2001), 21(6) ; 487-498. La kinase CDK2 et peut être CDK3 sont nécessaires pour la progression dans la phase G1 et l'entrée en phase S. Lors de la formation de complexe avec la cycline E, elles maintiennent l'hyperphosphorylation de pRb pour aider la progression de la phase G1 en phase S. Dans les complexes avec la Cycline A, CDK2 joue un rôle dans l'inactivation de E2F et est nécessaire pour la réalisation de la phase S (TD. Davies et al. (2001) Structure 9, 389-3).

Le complexe CDK1/cycline B régule la progression du cycle cellulaire entre la phase G2 et la phase M. La régulation négative du complexe CDK/Cycline B empêche les cellules normales d'entrer en phase S avant que la phase G2 ait été correctement et complètement achevée. (K.K. Roy and E.A. Sausville Current Pharmaceutical Design, 2001, 7, 1669-1687.

Un niveau de régulation de l'activité des CDK existe. Les activateurs de cycline dépendantes kinases (CAK) ont une action positive de régulation des CDK. CAK phosphoryle les CDK sur le résidu thréonine pour rendre l'enzyme cible totalement active.

La présence de défauts dans les molécules intervenant sur le cycle cellulaire entraîne l'activation des CDK et la progression du cycle, il est normal de vouloir inhiber l'activité des enzymes CDK pour bloquer la croissance cellulaire des cellules cancéreuses.

De nombreuses protéines impliquées dans la ségrégation des chromosomes et l'assemblage du fuseau ont été identifiées dans la levure et la drosophile. La désorganisation de ces protéines conduit à la non ségrégation des chromosomes et à des fuseaux monopolaires ou désorganisés. Parmi ces protéines, certaines kinases, dont **Aurora** et IpI1, provenant respectivement de drosophile et de S. *cerevisiae,* sont nécessaires pour la ségrégation des chromosomes et la séparation du centrosome. Un analogue humain de Ipl1 de levure a été récemment cloné et caractérisé par différents laboratoires. Cette kinase, nommée aurora2, STK15 ou BTAK appartient à la famille des kinases à sérine/thréonine. Bischoff et al. ont montré que Aurora2 est oncogène, et est amplifié dans les cancers colorectaux humains (EMBO J, 1998, 17, 3052-3065). Cela a également été exemplifié dans des cancers impliquant des tumeurs épithéliales telles que le cancer du sein.

### Définitions

Le terme « halogène » fait référence à un élément choisi parmi F, Cl, Br, et I.

Le terme « alkyle » fait référence à un substituant hydrocarboné saturé, linéaire ou ramifié, ayant de 1 à 12 atomes de carbone. Les substituants méthyle, éthyle, propyle, 1-méthyléthyl, butyle, 1-méthylpropyl, 2-méthylpropyle, 1,1-Diméthyléthyle, pentyle, 1-méthylbutyle, 2-méthylbutyle, 3-méthylbutyle, 1,1-Diméthylpropyle, 1,2-Diméthylpropyle, 2,2-Diméthylpropyle, 1-éthylpropyle, hexyle, 1-méthylpentyle, 2-méthylpentyle, 1-éthylbutyle, 2-éthylbutyle, 3,3-Diméthylbutyle, heptyle, 1-éthylpentyle, octyle, nonyle, décyle, undécyle, et dodécyle sont des exemples de substituant alkyle.

Le terme « alkylène » fait référence à un substituant hydrocarboné linéaire ou ramifié ayant une ou plusieurs insaturations, ayant de 2 à 12 atomes de carbone. Les substituants éthylènyle, 1-méthyléthylènyle, prop-1-ènyle, prop-2-ènyle, Z-1-méthylprop-1-ènyle, E-1-méthylprop-1-ènyle, Z-1,2-Diméthyl-prop-1-ènyle, E-1,2-Diméthylprop-1-ènyle, but-1,3-diényle, 1-méthylidènyl-prop-2-ènyle, Z-2-méthylbut-1,3-diényle, E-2-méthylbut-1,3-diényle, 2-méthyl-1-méthylidènylprop-2-ènyle, undéc-1-ènyle et undéc-10-ènyle sont des exemples de substituant alkylène.

Le terme « alkynyle » fait référence à un substituant hydrocarboné linéaire ou ramifié ayant au moins deux insaturations portées par une paire d'atomes de carbone vicinaux, ayant de 2 à 12 atomes de carbone. Les substituants éthynyle; prop-1-ynyle; prop-2-ynyle; et but-1-ynyle sont des exemples de substituant alkynyle.

Le terme « aryle » fait référence à un substituant aromatique mono- ou polycyclique ayant de 6 à 14 atomes de carbone. Les substituants phényle, napht-1-yle ; napht-2-yle ; anthracen-9-yl ; 1,2,3,4-tétrahydronapht-5-yle ; et 1,2,3,4-tétrahydronapht-6-yle sont des exemples de substituant aryle.

Le terme « hétéroaryle » fait référence à un substituant hétéroaromatique mono- ou polycyclique ayant de 1 à 13 atomes de carbone et de 1 à 4 hétéroatomes. Les substituants pyrrol-1-yle ; pyrrol2-yle ; pyrrol3-yle ; furyle ; thienyle ; imidazolyle ; oxazolyle ; thiazolyle ; isoxazolyle; isothiazolyle; 1,2,4-triazolyle ; oxadiazolyle ; thiadiazolyle ; tétrazolyle ; pyridyle ; pyrimidyle ; pyrazinyle ; 1,3,5-triazinyle ; indolyle ; benzo[b]furyle ; benzo[b]thiényle ; indazolyle ; benzimidazolyle ; azaindolyle ; quinoléyle ; isoquinoléyle ; carbazolyle ; et acridyle sont des exemples de substituant hétéroaryle.

Le terme « hétéroatome » fait référence ici à un atome au moins divalent, différent du carbone. N; O; S; et Se sont des exemples d'hétéroatome.

Le terme « cycloalkyle » fait référence à un substituant hydrocarboné cyclique saturé ou partiellement insaturé ayant de 3 à 12 atomes de carbone. Les substituants cyclopropyle; cyclobutyle; cyclopentyle; cyclopentènyle; cyclopentadiényle; cyclohexyle; cyclohexènyle; cycloheptyle; bicyclo[2.2.1]heptyle; cyclooctyle; bicyclo[2.2.2]octyle ; adamantyle ; et perhydronapthyle sont des exemples de substituant cycloalkyle.

Le terme « hétérocyclyle » fait référence à un substituant hydrocarboné cyclique saturé ou partiellement insaturé ayant de 1 à 13 atomes de carbone et de 1 à 4 hétéroatomes. De préférence, le substituant hydrocarboné cyclique saturé ou partiellement insaturé sera monocyclique et comportera 4 ou 5 atomes de carbone et 1 à 3 hétéroatomes.

Le terme « substitué » fait référence à un ou plusieurs substituants différents de H, par exemple halogène; alkyle; aryle; hétéroaryle, cycloalkyle ; hétérocyclyle ; alkylène; alkynyle; OH ; O-alkyle; O-alkylène ; O-aryle; O-hétéroaryle; NH₂ ; NH-alkyle; NH-aryle; NH-hétéroaryle; N-alkyle-alkyle' ; SH ; S-alkyle; S-aryle; S(O₂)H ; S(O₂)-alkyle; S(O₂)-aryle; SO₃H ; SO₃-alkyle; SO₃-aryle; CHO ; C(O)-alkyle; C(O)-aryle ; C(O)OH ; C(O)O-alkyle; C(O)O-aryle ; OC(O)-alkyle; OC(O)-aryle ; C(O)NH₂ ; C(O)NH-alkyle; C(O)NH-aryle ; NHCHO ; NHC(O)-alkyle; NHC(O)-aryle ; NH-cycloalkyle ; NH-hétérocyclyle.

Les produits selon l'invention peuvent être préparés à partir de méthodes conventionnelles de chimie organique. Le schéma 1 ci-dessous est illustratif de la méthode utilisée pour la préparation de l'exemple 1 concernant des 6-aza-indoles substitués. A ce titre, elle ne saurait constituer une limitation de la portée de l'invention, en ce qui concerne les méthodes de préparation des composés revendiqués. Préparation des dérivés de 6-aza-indole-2-carboxamide substitués en position 3 :

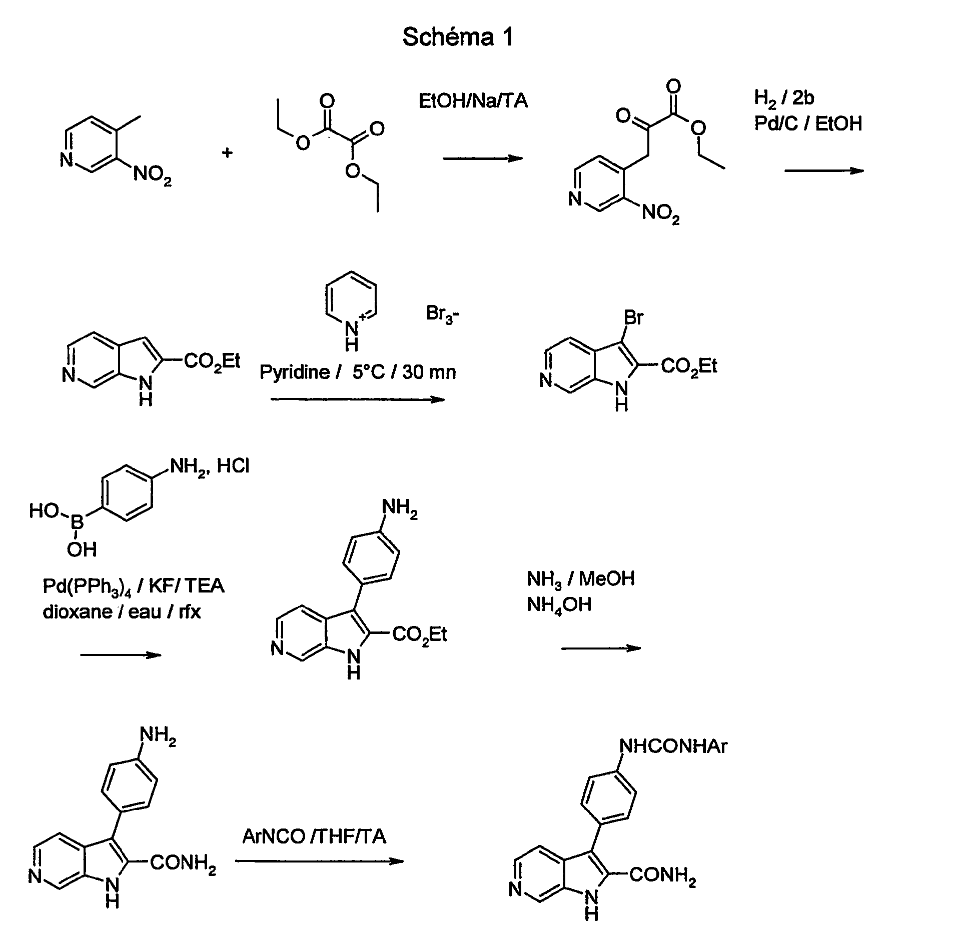

Le schéma 2 ci-dessous est illustratif de la méthode utilisée pour la préparation des exemples concernant les 7-aza-indoles substitués, en particulier l'exemple 7. A ce titre, elle ne saurait constituer une limitation de la portée de l'invention, en ce qui concerne les méthodes de préparation des composés revendiqués. Préparation des dérivés de 7-aza-indole-2-carboxamide substitués en position 3 :

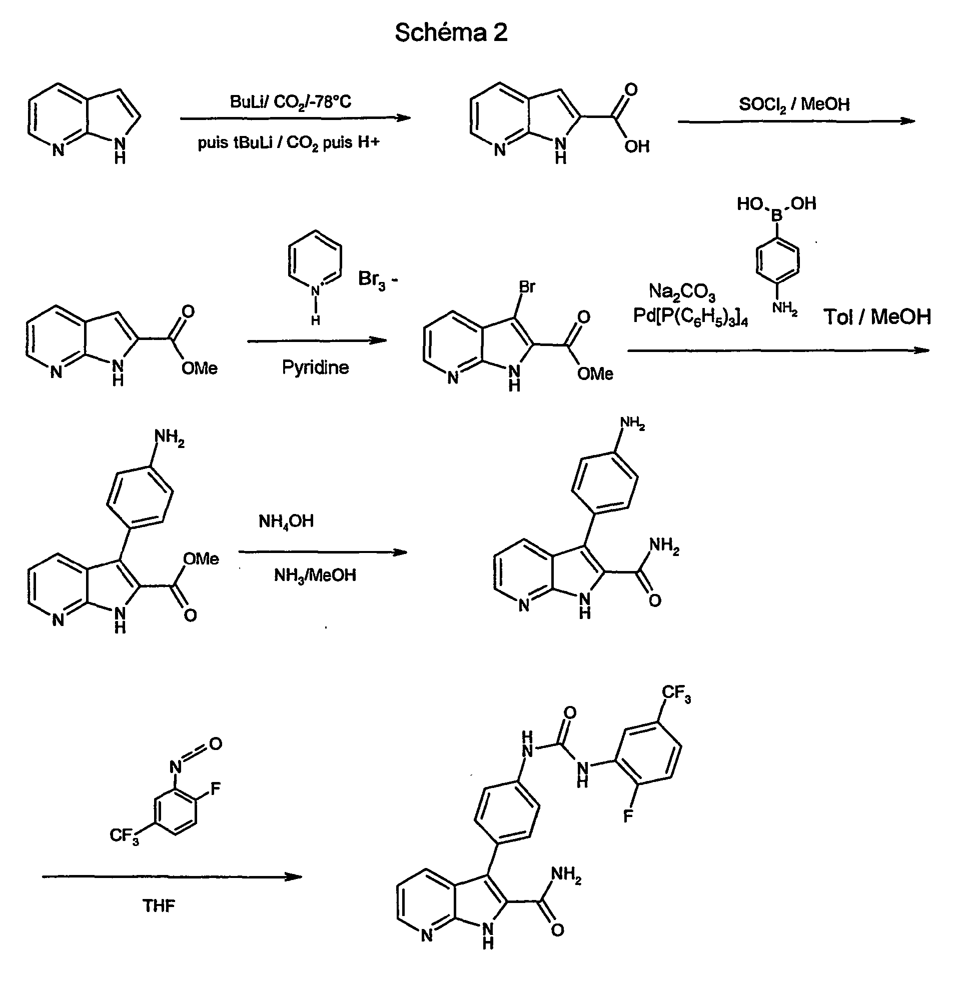

Les produits de formule générale (I) où Ra est différent de H peuvent être obtenus selon les méthodes conventionnelles connues de l'homme du métier, par exemple en remplaçant l'ammoniac dans l'aminolyse par l'alkylamine primaire correspondante.

La présente invention a encore pour objet un procédé de préparation des produits de formule (I) tels que définis précédemment, caractérisé en ce qu'un produit de formule générale (V) suivante : 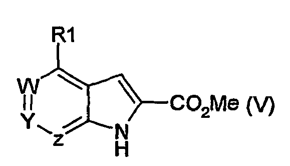 subit les étapes suivantes :
a) halogénation en position 3, puis
b) couplage de Suzuki en position 3, pour obtenir un produit de formule générale (III) suivante : 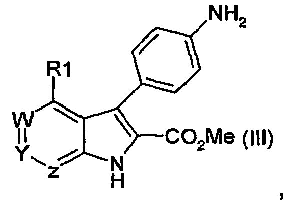 puis
c) amidation de l'ester en position 2 pour obtenir le produit de formule générale (II) suivante : 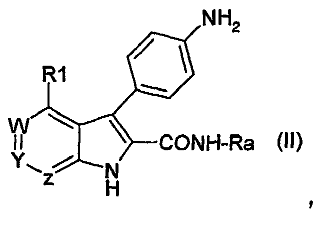 puis
d) acylation du groupe amino-phényle en position 3.

La présente invention a encore pour objet, à titre de produits intermédiaires, les composés de formule générale (II) suivante : 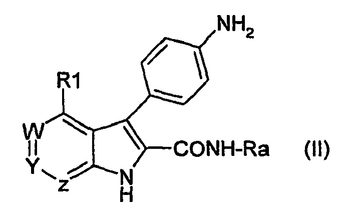 dans laquelle Z, Y, W sont tels que définis précédemment, pour la préparation des produits de formule générale (I).

Les analyses LC/MS ont été réalisées sur un appareil Micromass modèle LCT relié à un appareil HP 1100. L'abondance des produits a été mesurée à l'aide d'un détecteur à barrette de diodes HP G1315A sur une gamme d'onde de 200-600 nm et un détecteur à dispersion de lumière Sedex 65. L'acquisition des spectres de masses Mass spectra a été réalisée sur une gamme de 180 à 800. Les données ont été analysées en utilisant le logiciel Micromass MassLynx. La séparation a été effectuée sur une colonne Hypersil BDS C18, 3 µm (50 x 4.6 mm), en éluant par un gradient linéaire de 5 à 90% d'acétonitrile contenant 0,05% (v/v) d'acide trifluoroacétique (TFA) dans l'eau contenant 0,05% (v/v) TFA en 3,5 mn à un débit de 1 mL/mn. Le temps total d'analyse, incluant la période de rééquilibration de la colonne, est de 7 mn.

Les spectres MS ont été réalisés en électrospray (ES⁺) sur un appareil Platform II (Micromass). Les principaux ions observés sont décrits.

Les points de fusion ont été mesurés en capillaire, sur un appareil Mettler FP62, gamme 30°C à 300°C, montée de 2°C par minute.

### Purification par LC/MS:

Les produits peuvent être purifiés par LC/MS en utilisant un système Waters FractionsLynx composé d'une pompe à gradient Waters modèle 600, d'une pompe de régénération Waters modèle 515, d'une pompe de dilution Waters Reagent Manager, d'un auto-injecteur Waters modèle 2700, de deux vannes Rheodyne modèle LabPro, d'un détecteur à barrette de diodes Waters modèle 996, d'un spectromètre de masse Waters modèle ZMD et d'un collecteur de fractions Gilson modèle 204. Le système était contrôlé par le logiciel Waters FractionLynx. La séparation a été effectuée alternativement sur deux colonnes Waters Symmetry (C₁₈, 5µM, 19x50 mm, référence catalogue 186000210), une colonne étant en cours de régénération par un mélange eau/acétonitrile 95/5 (v/v) contenant 0,07% (v/v) d'acide trifluoroacétique, pendant que l'autre colonne était en cours de séparation. L'élution des colonnes a été effectuée en utilisant un gradient linéaire de 5 à 95% d'acétonitrile contenant 0,07% (v/v) d'acide trifluoroacétique dans l'eau, à un débit de 10 mL/mn. A la sortie de la colonne de séparation, un millième de l'effluent est séparé par un LC Packing Accurate, dilué à l'alcool méthylique à un débit de 0,5 mL/mn et envoyé vers les détecteurs, à raison de 75% vers le détecteur à barrette de diodes, et les 25% restants vers le spectromètre de masse. Le reste de l'effluent (999/1000) est envoyé vers le collecteur de fractions où le flux est éliminé tant que la masse du produit attendu n'est pas détectée par le logiciel FractionLynx. Les formules moléculaires des produits attendus sont fournies au logiciel FractionLynx qui déclenche la collecte du produit quand le signal de masse détecté correspond à l'ion [M+H]⁺ et/ou au [M+Na]⁺. Dans certains cas, dépendant des résultats de LC/MS analytique, quand un ion intense correspondant à [M+2H]⁺⁺ a été détecté, la valeur correspondant à la moitié de la masse moléculaire calculée (MW/2) est aussi fournie au logiciel FractionLynx. Dans ces conditions, la collecte est aussi déclenchée quand le signal de masse de l'ion [M+2H]⁺⁺ et/ou [M+Na+H]⁺⁺ sont détectés. Les produits ont été collectés en tube de verre tarés. Après collecte, les solvants ont été évaporés, dans un évaporateur centrifuge Savant AES 2000 ou Genevac HT8 et les masses de produits ont été déterminées par pesée des tubes après évaporation des solvants.

Un autre objet de l'invention se rapporte aux produits des exemples ci-dessous qui illustrent de façon non limitative la présente invention.

### Exemple 1 : 3-{4-[3-(2-Fluoro-5-trifluorométhyl-phényl)-uréido]-phényl}-1H-pyrrolo[2,3-c]pyridine-2-carboxamide

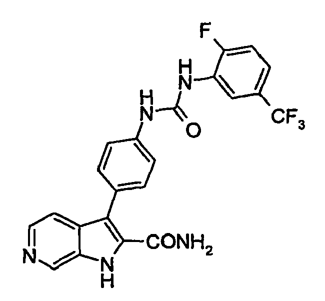

A une solution de 90 mg de 3-(4-aminophényl)-1 H-pyrrolo[2,3-c]pyridine-2-carboxamide dans 5 mL de tétrahydrofurane sont ajoutés, goutte à goutte, 50 µL de 2-fluoro-5-(trifluorométhyl)phénylisocyanate. Le mélange réactionnel est agité pendant 16 heures à température ambiante sous atmosphère d'argon puis concentré sous pression réduite. Le résidu obtenu est agité pendant 30 minutes dans 2 mL de dichlorométhane. Le solide en suspension est filtré et essoré. Après séchage sous vide, à 40°C, 115 mg de 3-{4-[3-(2-Fluoro-5-trifluorométhyl-phényl)-uréido]-phényl}-1H-pyrrolo[2,3-c]pyridine-2-carboxamide sont obtenus dont les caractéristiques sont les suivantes :
IR (KBr): 3455; 1661; 1602; 1542; 1444; 1341; 1312; 1127; 1070 et 819 cm⁻¹ R.M.N. ¹H: 6,98 (s large, 1H) ; 7,39 (m large, 1H) ; de 7,42 à 7,56 (m, 4H) ; 7,60 (d large, J = 8,0 Hz, 2H) ; 7,74 (s large, 1H) ; 8,17 (d, J = 6,0 Hz, 1H); 8,65 (d large, J = 7,5 Hz, 1 H) ; 8,82 (s, 1H) ; 8,94 (s large, 1H) ; 9,31 (s, 1H) ; 12,15 (s large, 1H).
Spectre de Masse (ES⁺) : m/z = 458 [M+H]⁺
Point de fusion: 286°C (Köfler).

### 3-(4-Aminophényl)-1H-pyrrolo[2,3-c]pyridine-2-carboxamide :

A une solution de 600 mg de 3-(4-aminophényl)-1H-pyrrolo[2,3-c]pyridine-2-carboxylate d'éthyle dans 62 mL de solution 3N d'ammoniac dans le méthanol sont ajoutés 11 mL de solution aqueuse d'ammoniaque à 22%. Le mélange réactionnel est agité pendant 20 heures dans un autoclave à 80°C (12 bars), puis concentré sous pression réduite. Le résidu obtenu est dilué dans 100 mL de méthanol, traité au noir de charbon et chauffé au reflux pendant 30 minutes. Le mélange est filtré à chaud sur célite puis rincé avec 2 x 10 mL de méthanol. Le filtrat est concentré sous pression réduite pour donner 490 mg de 3-(4-aminophényl)-1 H-pyrrolo[2,3-c]pyridine-2-carboxamide sous forme de meringue et dont les caractéristiques sont les suivantes :
Spectre de Masse (EI) m/z = 252 [M]^{+°}, m/z = 235 [M-NH₃]^{+°}

### 3-(4-Aminophényl)-1 H-pyrrolo[2,3-c]pyridine-2-carboxylate d'éthyle :

A une solution de 1 g de 3-bromo-1H-pyrrolo[2,3-c]pyridine-2-carboxylate d'éthyle dans 100 mL de dioxanne, sont ajoutés 773 mg de chlorhydrate d'acide 4-aminophényl-boronique, 1,1 g de fluorure de potassium dans 9 mL d'eau. Le mélange réactionnel est agité sous atmosphère d'argon pendant 15 minutes. 425 mg de tétrakis (triphénylphosphine) palladium (0) et 630 µL de triéthylamine sont ajoutés. Le mélange réactionnel est agité pendant 17 heures au reflux. Après traitement au noir de charbon puis filtration sur célite®, le filtrat est concentré sous pression réduite. Le résidu est purifié par flash-chromatographie sur colonne de silice (60 ; 35-70 µM), en éluant par un mélange de dichlorométhane, méthanol et acétonitrile (90/5/5 en volumes). 600 mg de 3-(4-aminophényl)-1 H-pyrrolo[2,3-c]pyridine-2-carboxylate d'éthyle sont obtenus, dont les caractéristiques sont les suivantes :
Spectre de Masse (EI) m/z =281 [M]^{+°}, m/z = 235 [M-OEt]^{+°}

### 3-Bromo-1 H-pyrrolo[2,3-c]pyridine-2-carboxylate d'éthyle :

A une solution de 2,24 g de 1H-pyrrolo[2,3-c]pyridine-2-carboxylate d'éthyle dans 150 mL de pyridine, sont ajoutés, goutte à goutte, une solution de 3,53g de tribromure de pyridinium dans 30 mL de pyridine, à 5°C. Le mélange réactionnel est ensuite agité pendant 16 heures à une température voisine de 20°C puis lavé avec 500 mL d'eau glacée. La suspension est filtrée. Le solide résultant est lavé à l'eau puis séché à l'étuve sous vide à 40°C. 1,97 g de 3-bromo-1 H-pyrrolo[2,3-c]pyridine-2-carboxylate d'éthyle sont obtenus, dont les caractéristiques sont les suivantes:
Spectre de Masse (EI) m/z = 269 [M]^{+°}, m/z = 189 [M-Br]^{+°}, m/z = 144 [M-OEt]^{+°}

### 1H-Pyrrolo[2,3-c]pyridine-2-carboxylate d'éthyle :

On charge dans un autoclave 1,8 g de palladium sur carbone à 10 %, puis on inerte avec un courrant d'argon. Une solution de 6 g de 3-(3-nitropyridin-4-yl)-2-oxo-propionate d'éthyle dans 72 mL d'éthanol absolu est ajoutée. Le milieu réactionnel est ensuite agité 3 heures à 20 °C sous une pression de 2 bars d'hydrogène. Le mélange est alors filtré sur célite®. Le filtrat est concentré sous pression réduite, séché à l'étuve à 40°C pour donner 4g de 1 H-pyrrolo[2,3-c]pyridine-2-carboxylate d'éthyle dont les caractéristiques sont les suivantes:
Spectre de Masse (EI) m/z = 190 [M^{+°}], m/z = 144 [M-OEt]^{+°}

### 3-(3-Nitropyridin-4-yl)-2-oxo-propionate d'éthyle :

A une solution de 930 mg de sodium dans 50 mL d'éthanol absolu, sont ajoutés rapidement 26 mL de diéthyle oxalate. Le milieu réactionnel est agité 15 minutes à 20°C. Une solution de 3,8 g de 4-méthyl-3-nitropyridine dans 50 mL d'éthanol absolu est alors ajoutée, goutte à goutte, en 1 heure. Le mélange réactionnel est agité pendant 4 heures à une température voisine de 20°C, puis concentré sous pression réduite. Le résidu est repris par 100 mL d'éther éthylique puis filtré. Le solide est agité avec 40 mL d'acide chlorhydrique 5N puis filtré, lavé à l'eau et séché sous vide à 40°C pour donner 6,2 g de 3-(3-nitropyridin-4-yl)-2-oxo-propionate d'éthyle avec les caractéristiques suivantes :
Spectre de Masse (EI) m/z = 238 [M^{+°}].

### Exemple 2 : 3-{4-[3-(2-Méthoxy-5-Méthyl-phényl)-uréido]-phényl}-1H-pyrrolo[2,3-c]pyridine-2-carboxamide

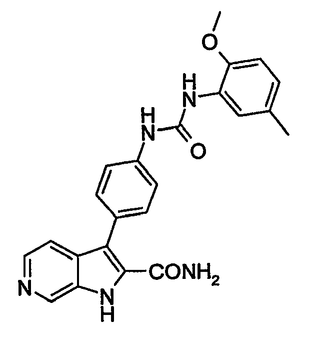

A une solution de 100 mg de 3-(4-aminophényl)-1H-pyrrolo[2,3-c]pyridine-2-carboxamide dans 5 mL de tétrahydrofurane sont ajoutés, goutte à goutte, 54,4 µL de 2-méthoxy-5-méthylphénylisocyanate. Le mélange réactionnel est agité pendant 16 heures à température ambiante sous atmosphère d'argon puis concentré sous pression réduite. Le résidu obtenu est agité pendant 30 minutes dans 2 mL de dichlorométhane. Le solide en suspension est filtré, lavé à l'eau et essoré. Après séchage sous vide, à 40°C, 40 mg de 3-{4-[3-(2-méthoxy-5-Méthyl-phényl)-uréido]-phényl}-1H-pyrrolo[2,3-c]pyridine-2-carboxamide sont obtenus dont les caractéristiques sont les suivantes :
IR (KBr), 3458; 3331; 1664; 1595; 1537; 1315; 1285; 1213; 1135; 1033 cm⁻¹
R.M.N. ¹H: 2,24 (s, 3H) ; 3,86 (s, 3H) ; 6,75 (d large, J = 8,5 Hz, 1H) ; de 6,85 à 6,95 (m, 2H) ; 7,43 (d large, J = 8,5 Hz, 2H) ; 7,46 (d, J = 5,5 Hz, 1 H) ; 7,58 (d large, J = 8,5 Hz, 2H) ; 7,73 (s large, 1H) ; 8,02 (s large, 1H) ; 8,16 (d, J = 5,5 Hz, 1H) ; 8,22 (s, 1H) ; 8,82 (s, 1H) ; 9,44 (s large, 1H) ; 12,1 (s large, 1 H).
Spectre de Masse (EI) : m/z = 415 [M^{+°}]
Point de fusion: 227°C

### Exemple 3 : Trifluoroacétate de 3-{4-[3-(3-chloro-phényl)-uréido]-phényl}-1H-pyrrolo[2,3-c]pyridine-2-carboxamide,.

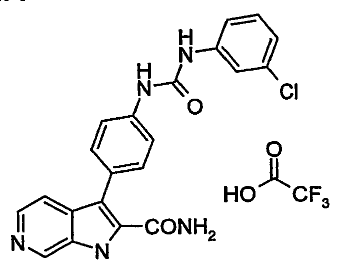

A une solution de 100 mg de 3-(4-aminophényl)-1H-pyrrolo[2,3-c]pyridine-2-carboxamide dans 5 mL de tétrahydrofurane sont ajoutés, goutte à goutte, 45,2 µL de 3-chlorophénylisocyanate. Le mélange réactionnel est agité pendant 16 heures à température ambiante sous atmosphère d'argon puis concentré sous pression réduite. Le résidu obtenu est agité pendant 30 minutes dans 2 mL de dichlorométhane. Le solide en suspension est filtré, lavé à l'eau et essoré. La purification finale est réalisée par LC/MS préparative pour donner après séchage sous vide à 40°C 70 mg de 3-{4-[3-(3-chloro-phényl)-uréido]-phényl)-1H-pyrrolo[2,3-c]pyridine-2-carboxamide sous forme de sel de trifluoroacétate et dont les caractéristiques sont les suivantes :
IR (KBr): 3390; 1672; 1592; 1537; 1483; 1203; 1138; 836; 722 cm⁻¹
R.M.N. ¹H: 7,03 (m, 1H) ; de 7,26 à 7,34 (m, 2H) ; de 7,42 à 7,52 (m, 3H) ; 7,63 (d large, J = 8,5 Hz, 2H) ; 7,74 (s large, 1H) ; 7,97 (d, J = 6,0 Hz, 1H) ; 8,06 (s large, 1H) ; 8,31 (d, J = 6,0 Hz, 1H) ; 9,03 (s large, 2H) ; 9,13 (s, 1H) ; 13,35 (m étalé, 1H) .
Spectre de Masse (ES⁺) : m/z = 406 [MH⁺]
Point de fusion: 221 °C

### Exemple 4: Trifluoroacetate de 3-{4-[3-(3-chloro-4-fluoro-phényl)-uréido]-phényl}-1H-pyrrolo[2,3-c]pyridine-2-carboxamide

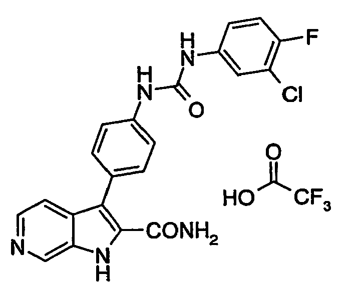

A une solution de 100 mg de 3-(4-aminophényl)-1H-pyrrolo[2,3-c]pyridine-2-carboxamide dans 5 mL de tétrahydrofurane sont ajoutés, goutte à goutte, 46,2 µL de 3-chloro-4-fluorophénylisocyanate. Le mélange réactionnel est agité pendant 16 heures à température ambiante sous atmosphère d'argon puis concentré sous pression réduite. Le résidu obtenu est agité pendant 30 minutes dans 2 mL de dichlorométhane. Le solide en suspension est filtré, lavé à l'eau et essoré. La purification finale est réalisée par LC/MS préparative pour donner après séchage sous vide, à 40°C 105 mg de 3-{4-[3-(3-chloro-4-fluoro-phényl)-uréido]-phényl}-1H-pyrrolo[2,3-c]pyridine-2-carboxamide sous forme de sel de trifluoroacétate et dont les caractéristiques sont les suivantes :
IR (KBr) : 3452; 1673; 1601; 1544; 1500; 1208; 1143; 836; 803; 722 cm⁻¹
R.M.N. ¹H : De 7,32 à 7,38 (m, 2H) ; de 7,44 à 7,54 (m, 3H) ; 7,64 (d large, J = 8,5 Hz, 2H) ; 7,84 (d large, J = 7,5 Hz, 1H) ; 8,01 (d, J = 6,0 Hz, 1H) ; 8,09 (s large, 1H) ; 8,32 (d, J = 6,0 Hz, 1H) ; 9,10 (s large, 2H) ; 9,16 (s, 1H) ; 13,4 (m étalé, 1H).
Spectre de Masse (ES⁺) : m/z = 424 [MH⁺]
Point de fusion: 214°C

### Exemple 5 : Trifluoroacétate de 3-{4-[3-(2-fluoro-5-Méthyl-phényl)-uréido]-phényl}-1H-pyrrolo[2,3-c]pyridine-2-carboxamide

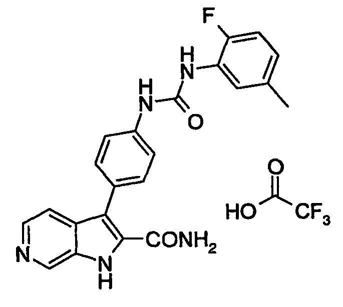

A une solution de 100 mg de 3-(4-aminophényl)-1H-pyrrolo[2,3-c]pyridine-2-carboxamide dans 5 mL de tétrahydrofurane sont ajoutés, goutte à goutte, 48,3 µL de 2-fluoro-5-méthylphénylisocyanate. Le mélange réactionnel est agité pendant 16 heures à température ambiante sous atmosphère d'argon puis concentré sous pression réduite. Le résidu obtenu est agité pendant 30 minutes dans 2 mL de dichlorométhane. Le solide en suspension est filtré, lavé à l'eau et essoré. La purification finale est réalisée par LC/MS préparative pour donner après séchage sous vide, à 40°C, 36 mg de 3-{4-[3-(2-fluoro-5-Méthyl-phényl)-uréido]-phényl}-1H-pyrrolo[2,3-c]pyridine-2-carboxamide sous forme de sel de trifluoroacétate dont les caractéristiques sont les suivantes :
IR (KBr) : 3452; 1675; 1603; 1544;1314; 1202; 1144; 836; 805; 722 cm⁻¹
R.M.N. ¹H : 2,29 (s, 3H) ; 6,82 (m, 1H) ; 7,12 (dd, J = 8,5 et 11,5 Hz, 1H) ; de 7,46 à 7,51 (m, 3H) ; 7,62 (d large, J = 8,5 Hz, 2H) ; de 7,97 à 8,03 (m, 2H) ; 8,08 (s large, 1 H) ; 8,32 (d, J = 6,5 Hz, 1 H) ; 8,54 (d large, J = 2,5 Hz, 1H) ; 9,15 (s, 1H) ; 9,25 (s, 1H) ; 13,4 (m étalé, 1 H)
Spectre de Masse (ES⁺) : m/z = 404 [MH⁺]
Point de fusion: 222°C

### Exemple 6: Trifluoroacétate de 3-[4-(3-m-tolyl-uréido)-phényl]-1H-pyrrolo[2,3-c]pyridine-2-carboxamide

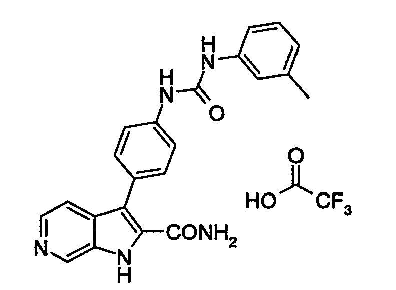

A une solution de 100 mg de 3-(4-aminophényl)-1H-pyrrolo[2,3-c]pyridine-2-carboxamide dans 5 mL de tétrahydrofurane sont ajoutés, goutte à goutte, 47,8 µL de m-tolylisocyanate. Le mélange réactionnel est agité pendant 16 heures à température ambiante sous atmosphère d'argon puis concentré sous pression réduite. Le résidu obtenu est agité pendant 30 minutes dans 2 mL de dichlorométhane. Le solide en suspension est filtré, lavé à l'eau et essoré. La purification finale est réalisée par LC/MS préparative pour donner après séchage sous vide, à 40°C, 40 mg de 3-[4-(3-m-tolyl-uréido)-phényl]-1H-pyrrolo[2,3-c]pyridine-2-carboxamide sous forme de sel de trifluoroacétate dont les caractéristiques sont les suivantes :
IR (KBr) : 3408; 1699; 1595; 1526; 1203; 1138; 834; 797; 724 cm⁻¹
R.M.N. ¹H: 2,29 (s, 3H) ; 6,81 (d large, J = 7,5 Hz, 1H) ; 7,17 (t, J = 7,5 Hz, 1 H) ; 7,25 (d large, J = 7,5 Hz, 1H) ; 7,32 (s large, 1 H) ; 7,44 (s large, 1H) ; 7,47 (d large, J = 8,5 Hz, 2H) ; 7,62 (d large, J = 8,5 Hz, 2H) ; 7,96 (m large, 1H) ; 8,06 (s large, 1H) ; 8,30 (d, J = 6,0 Hz, 1H) ; 8,67 (s, 1H) ; 8,86 (s, 1H) ; 9,12 (s, 1H) ; 13,3 (m étalé, 1 H).
Spectre de Masse (ES⁺) : m/z = 386 [MH⁺]

### Exemple 7: 3-{4-[3-(2-Fluoro-5 trifluorométhyl-phényl)-uréido]-phényl}-1H-pyrrolo[2,3-b]pyridine-2-carboxamide

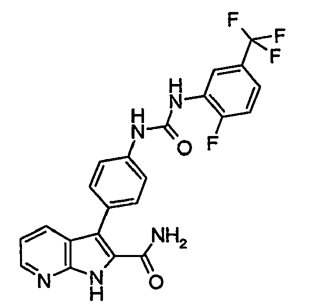

A une solution de 130 mg de 3-(4-aminophényl)-1H-pyrrolo[2,3-b]pyridine-2-carboxamide dans 5 mL de tétrahydrofurane sont ajoutés, goutte à goutte, 85 µL de 2-fluoro-5-(trifluorométhyl)phénylisocyanate. Le mélange réactionnel est ensuite agité pendant 16 heures à température ambiante sous atmosphère d'argon puis concentré sous pression réduite. Le résidu obtenu est chromatographié sur colonne de silice (éluant dichlorométhane-méthanol 9-1 en volumes). Les fractions contenant l'attendu sont concentrées sous pression réduite. On obtient 237 mg de 3-{4-[3-(2-fluoro-5-trifluorométhyl-phényl)-uréido]-phényl}-1H-pyrrolo[2,3-b]pyridine-2-carboxamide sous la forme d'un solide blanc dont les caractéristiques sont les suivantes :
IR (KBr) : 1659; 1623; 1542; 1443; 1339; 1316; 1119 cm⁻¹
R.M.N. ¹H : 7,08 (m étalé, 1H) ; 7,14 (dd, J = 5,0 et 8,0 Hz, 1H) ; 7,40 (m, 1H) ; 7,46 (d large, J = 8,5 Hz, 2H) ; 7,51 (m, 1H) ; 7,57 (d large, J = 8,5 Hz, 2H) ; de 7,55 à 7,60 (m masqué, 1H) ; 7,92 (d large, J = 8,0 Hz, 1H) ; 8,38 (d large, J = 5,0 Hz, 1 H) ; 8,64 (d large, J = 7,5 Hz, 1H) ; 9,01 (s large, 1H) ; 9,36 (s large, 1H) ; 12,1 (s large, 1H) .
Spectre de Masse (ES⁺) : m/z = 458 [M+H⁺]
Point de fusion: 232°C (Köfler).

### 3-(4-Aminophényl)-1H-pyrrolo[2,3-b]pyridine-2-carboxamide :

A une solution de 260 mg de 3-(4-aminophényl)-1H-pyrrolo[2,3-b]pyridine-2-carboxylate de méthyle dans 30 mL de solution 7N d'ammoniac dans le méthanol sont ajoutés 5 mL de solution aqueuse d'ammoniaque à 22%. Le mélange réactionnel est ensuite agité pendant 20 heures dans un autoclave à 80°C (12.6 bars), puis concentré sous pression réduite. Le résidu obtenu est chromatographié sur colonne de silice (éluant dichlorométhane-méthanol 9-1 en volumes ). Les fractions contenant l'attendu sont concentrées sous pression réduite. On obtient 140 mg de 3-(4-aminophényl)-1H-pyrrolo[2,3-b]pyridine-2-carboxamide sous forme d'un solide jaune pâle dont les caractéristiques sont les suivantes :
Point de fusion : 139°C

### 3-(4-Aminophényl)-1H-pyrrolo[2,3-b]pyridine-2-carboxylate de méthyle :

A une solution de 0.64 g de 3-bromo-1 H-pyrrolo[2,3-b]pyridine-2-carboxylate de méthyle dans 50 mL de toluène et 50 ml de méthanol, sont ajoutés 1.08 g de chlorhydrate d'acide 4-aminophényl-boronique et 0.9 ml de triéthylamine. Le mélange réactionnel est ensuite agité sous atmosphère d'argon pendant 15 minutes. 144 mg de tétrakis(triphénylphosphine)-palladium(0), 0.3 g de chlorure de lithium, 0.66g de carbonate de sodium, et 7.5 mL d'eau distillée sont ajoutés successivement. Le mélange réactionnel est agité pendant 8 heures au reflux. Après filtration sur célite®, le filtrat est concentré sous pression réduite. Le résidu est purifié par chromatographie sur colonne de silice en éluant par un mélange d'acétate d'éthyle et cyclohexane (7-3 en volumes). 400 mg de 3-(4-aminophényl)-1H-pyrrolo[2,3-b]pyridine-2-carboxylate de méthyle sont obtenus sous la forme d'un solide jaune, dont les caractéristiques sont les suivantes :
Point de fusion : 236°C

### 3-Bromo-1H-pyrrolo[2,3-b]pyridine-2-carboxylate de méthyle :

A une solution de 3.2 g de chlorhydrate de 1H-pyrrolo[2,3-b]pyridine-2-carboxylate de méthyle dans 165 mL de pyridine, on ajoute goutte à goutte à 0°C sous atmosphère d'argon une solution de 5.04 g de tribromure de pyridinium dans 35 mL de pyridine. Le mélange réactionnel est ensuite agité à 0°C, puis coulé sur un mélange de 250 g de glace pilée et 750 ml d'eau distillée. La suspension est filtrée, le solide est lavé par 2 fois 25 mL d'eau distillée, puis séché à l'air libre. On obtient 0.87 g de 3-bromo-1 H-pyrrolo[2,3-b]pyridine-2-carboxylate de méthyle sous la forme d'un solide beige, dont les caractéristiques sont les suivantes :
Spectre de Masse (ES⁺) : m/z = 256 [M+H⁺]

### Chlorhydrate de 1 H-pyrrolo[2,3-b]pyridine-2-carboxylate de méthyle :

A une solution de 4 g de chlorhydrate d'acide 1 H-pyrrolo[2,3-b]pyridine-2-carboxylique dans 100 mL de méthanol on ajoute goutte à goutte 6 mL de chlorure de thionyle à température ambiante. Le mélange réactionnel est ensuite agité pendant 5 heures à température ambiante, puis concentré sous pression réduite. Le résidu obtenu est trituré dans 50 mL d'éther éthylique, puis séché sous vide à 40°C. On obtient 3.22g de chlorhydrate de 1 H-pyrrolo[2,3-b]pyridine-2-carboxylate de méthyle sous la forme d'un solide jaune pâle utilisé tel que dans l'étape suivante.

### Chlorhydrate d'acide 1H-pyrrolo[2,3-b]pyridine-2-carboxylique

A une solution refroidie à -70°C de 6.03 g de 1H-pyrrolo[2,3-b]pyridine dans 75 ml de THF anhydre on ajoute goutte à goutte 33mL de solution 1.6M de n-butyllithium dans l'hexane. Après 15 minutes d'agitation à -70°C on ajoute à la solution 20g de carboglace en morceaux. On laisse ensuite revenir à température ambiante, puis on concentre sous pression réduite. On obtient 8.4g d'un solide blanc qui est dissout dans 175 mL de tétrahydrofurane. Cette solution est refroidie à -70°C, puis on ajoute goutte à goutte 35 mL de solution 1.5M de t-butyllithium dans l'hexane. Après 30 min d'agitation à - 70°C, on ajoute 20 g de carboglace en morceaux dans la solution. On laisse ensuite revenir à température ambiante, puis on coule ce mélange réactionnel sur 50 mL d'eau distillée refroidie à 0°C. Le tétrahydrofurane est évaporé sous pression réduite. La solution aqueuse résiduelle est diluée par 150 mL d'eau distillée, lavée deux fois par 100 mL de dichlorométhane, acidifiée à pH1 par ajout de 30 mL de d'une solution aqueuse 5N d'acide chlorhydrique, puis concentrée sous pression réduite. On obtient 10.01 g de solide pâteux qu'on recristallise dans 50 mL de méthanol. Le solide obtenu est traité par un mélange de 50 mL d'isopropanol chlorhydrique 7N et 50 mL d'éther isopropylique. Après séchage à l'air, on obtient 5.71 g de chlorhydrate d'acide 1H-pyrrolo[2,3-b]pyridine-2-carboxylique sous la forme d'un solide crème.
Spectre de Masse (EI) : m/z = 162 [M^{+°}]

### Exemple 8: 3-{4-[3-(2-Fluoro-5-trifluorométhyl-phényl)-uréido]-phényl}-7-oxy-1H-pyrrolo[2,3-b]pyridine-2-carboxamide

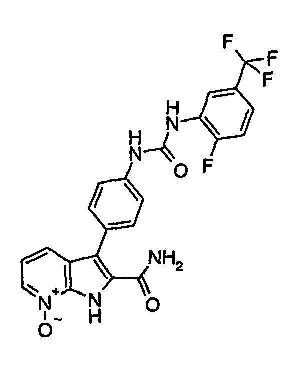

A une solution maintenue à 0°C de 50 mg de 3-{4-[3-(2-fluoro-5-trifluorométhyl-phényl)-uréido]-phényl}-1H-pyrrolo[2,3-b]pyridine-2-carboxamide dans 2 mL de chloroforme on ajoute goutte à goutte 0.31 mL d'une solution de 0.7M d'acide métachloroperbenzoique dans le chloroforme. La solution est agitée à 0°C pendant 4 heures puis à température ambiante pendant 16 heures. Le mélange réactionnel est dilué par 3 mL de dichlorométhane, filtré sur verre fritté N° 4, le solide obtenu est lavé deux fois par 3 mL de dichlorométhane, puis séché à l'air. On obtient 40 mg de 3-{4-[3-(2-fluoro-5-trifluorométhyl-phényl)-uréido]-phényl}-7-oxy-1 H-pyrrolo[2,3-b]pyridine-2-carboxamide sous la forme d'un solide jaune pâle dont les caractéristiques sont les suivantes :
IR (KBr) : 3352; 1671; 1609; 1545; 1442; 1340; 1315; 1239; 1119; 1069 et 885 cm⁻¹
R.M.N. ¹H : 7,16 (m, 1H) ; de 7,35 à 7,58 (m, 7H) ; 7,63 (m étalé, 1H) ; 7,77 (m étalé, 1H) ; 8,31 (d large, J = 6,0 Hz, 1H) ; 8,65 (d large, J = 8,5 Hz, 1H) ; 8,94 (s large, 1H) ; 9,29 (s, 1H) ; de 12,5 à 13,2 (m très étalé, 1H).
Spectre de masse (ES⁺) : m/z = 474 [M+H⁺]
Point de fusion: 220°C (Köfler).

### Exemple 9 : 3-{4-[3-(2-Fluoro-phényl)-uréido]-phényl}-1H-pyrrolo[2,3-b]pyridine-2-carboxamide

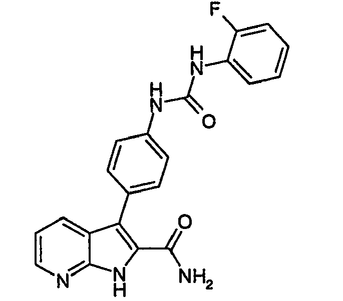

66.6 mg de solide beige de 3-{4-[3-(2-Fluoro-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 2-Fluoro-phénylisocyanate.
Point de fusion = 268.7°C (Büchi)
Spectre de masse (ES⁺) : [M + H]⁺ = 390
Temps de rétention (min) : 3.71

### Exemple 10 : 3-{4-[3-(2-Méthoxy-phényl)-uréido]-phényl}-1H-pyrrolo[2,3-b]pyridine-2-carboxamide

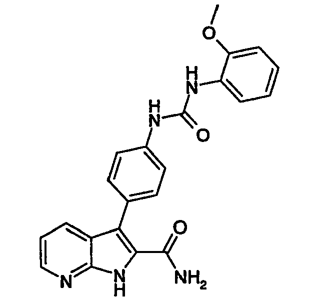

83.6 mg de solide beige de 3-{4-[3-(2-Méthoxy-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 2-Méthoxy-phénylisocyanate.
Point de fusion : 227.1 °C (Büchi)
Spectre de masse (ES⁺) : [M + H]⁺ = 402
Temps de rétention (min) : 3.77

### Exemple 11 : 3-{4-[3-(4-Trifluorométhyl-phényl)-uréido]-phényl}-1H-pyrrolo[2,3-b]pyridine-2-carboxamide

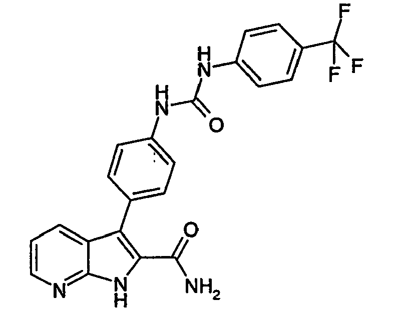

77.6 mg de solide blanc de 3-{4-[3-(4-Triflurométhyl-phényl)-uréido]-phényl}1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 4-trifluorométhyl-phénylisocyanate.
Point de fusion : 296.2 °C (Büchi)
Spectre de masse (ES⁺) : [M + H]⁺ = 440
Temps de rétention (min) : 4.24

### Exemple 12 : 3-{4-[3-(2-Chloro-5-trifluorométhyl-phényl)-uréido]-phényl)-1H-pyrrolo[2,3-b]pyridine-2-carboxamide.

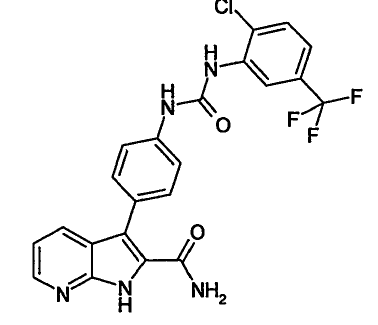

40.56 mg de solide blanc de 3-{4-[3-(2-Chloro-5-trifluorométhyl-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 2-Chloro-5-trifluorométhyl-phénylisocyanate.
Point de fusion: 188.3°C (Büchi)
Spectre de masse (ES⁺) : [M + H]⁺ = 474
Temps de rétention (min) : 4.51

### Exemple 13 : 3-{4-[3-(2-Fluoro-3-trifluorométhyl-phényl)-uréido]-phényl)-1H-pyrrolo[2,3-b]pyridine-2-carboxamide.

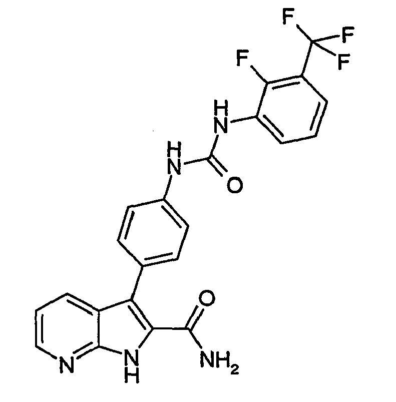

79 mg de solide blanc de 3-{4-[3-(2-Fluoro-3-trifluorométhyl-phényl)-uréido]-phényl}-1H-pyrrolo[2,3-b]pyridine-2-carboxamide sont préparés comme décrit_dans l'exemple 7 à partir de 3-(4-Aminophényl)-1H-pyrrolo[2,3-b]pyridine-2-carboxamide et de 2-Fluoro-3-trifluorométhyl-phénylisocyanate.
Point de fusion : 265.4°C (Büchi)
Spectre de masse (ES⁺) : [M + H]⁺ = 458
Temps de rétention (min) : 4.24

### Exemple 14: 3-{4-[3-(4-Fluoro-3-trifluorométhyl-phényl)-uréido]-phényl}-1H-pyrrolo[2,3-b)pyridine-2-carboxamide.

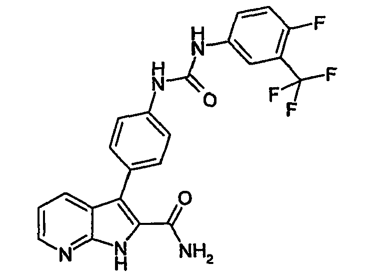

76.5 mg de solide brun de 3-{4-[3-(4-Fluoro-3-trifluorométhyl-phényl)-uréido]-phényl}-1H-pyrrolo[2,3-b]pyridine-2-carboxamide sont préparés comme décrit_dans l'exemple 7 à partir de 3-(4-Aminophényl)-1H-pyrrolo[2,3-b]pyridine-2-carboxamide et de 4-Fluoro-3-trifluorométhyl-phénylisocyanate.
Point de fusion: 234.7°C (Büchi)
Spectre de masse (ES⁺) : [M + H]⁺ = 458
Temps de rétention (min) :4.22

### Exemple 15 : 3-{4-[3-(3-Fluoro-5-trifluorométhyl-phényl)-uréido]-phényl}-1H-pyrrolo[2,3-b]pyridine-2-carboxamide

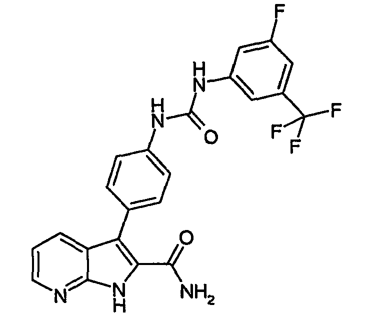

78.1 mg de solide beige de 3-{4-[3-(3-Fluoro-5-trifluorométhyl-phényl)-uréido]-phényl}-1H-pyrrolo[2,3-b]pyridine-2-carboxamide sont préparés comme décrit_dans l'exemple 7 à partir de 3-(4-Aminophényl)-1H-pyrrolo[2,3-b]pyridine-2-carboxamide et de 3-Fluoro-5-trifluorométhyl-phénylisocyanate.
Point de fusion: 257.5 °C (Büchi)
Spectre de masse (ES⁺) : [M + H]⁺ = 458
Temps de rétention (min) : 4.42

### Exemple 16: 3-{4-[3-(4-TrifluoroMéthoxy-phényl)-uréidol-phényl}-1H-pyrrolo[2,3-b]pyridine-2-carboxamide.

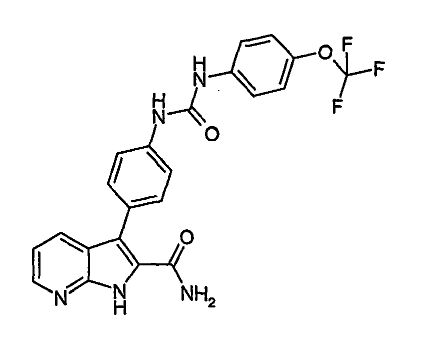

92.3 mg de poudre marron de 3-{4-[3-(4-TrifluoroMéthoxy-phényl)-uréido]-phényl}-1H-pyrrolo[2,3-b]pyridine-2-carboxamide sont préparés comme décrit_dans l'exemple 7 à partir de 3-(4-Aminophényl)-1H-pyrrolo[2,3-b]pyridine-2-carboxamide et de 4-Trifluorométhoxy-phénylisocyanate.
Point de fusion: 258.9°C (Büchi)
Spectre de masse (ES⁺) : [M + H]⁺ = 456
Temps de rétention (min) : 4.29

### Exemple 17 : 3-{4-[3-(3,4-Diméthoxy-phényl)-uréido]-phényl}-1H-pyrrolo[2,3-b]pyridine-2-carboxamide

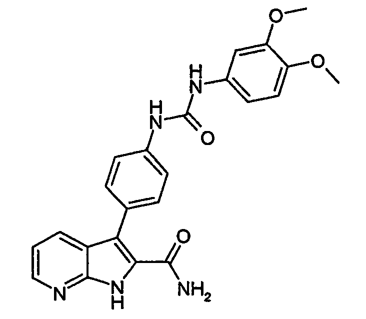

79 mg de solide beige de 3-{4-[3-(3,4-Diméthoxy-phényl)-uréido]-phényl}-1 H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et 3,4-Diméthoxy-phénylisocyanate.
Point de fusion : 223.7°C (Büchi)
Spectre de masse (ES⁺) : [M + H]⁺ = 432
Temps de rétention (min) : 3.27

### Exemple 18 : 3-{4-[3-(2,5-Diméthyl-phényl)-uréido]-phényl}-1H-pyrrolo[2,3-b]pyridine-2-carboxamide

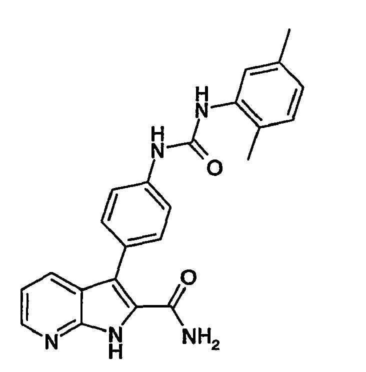

75.9 mg de solide blanc de 3-{4-[3-(2,5-Diméthyl-phényl)-uréido]-phényl}-1 H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 2,5-Diméthyl-phénylisocyanate.
Point de fusion : 308.8 °C (Büchi)
Spectre de masse (ES⁺) : [M+H]⁺ = 400
Temps de rétention (min) : 3.90

### Exemple 19: 3-{4-[3-(3-Méthoxy-phényl)-uréido]-phényl}-1H-pyrrolo[2,3-b]pyridine-2-carboxamide

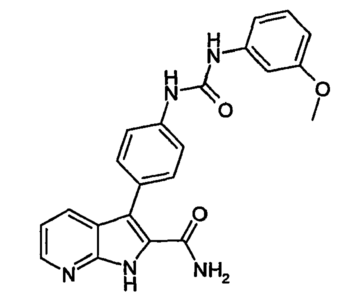

55.5 mg de solide beige de 3-{4-[3-(3-Méthoxy-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 3-Méthoxy-phénylisocyanate.
Point de fusion : 306.2 °C (Büchi)
Spectre de masse (ES⁺) : [M+H]⁺ = 402
Temps de rétention (min) : 3.39

### Exemple 20 : 3-{4-[3-(3-Trifluorométhyl-phényl)-uréido]-phényl}-1H-pyrrolo[2,3-b]pyridine-2-carboxamide

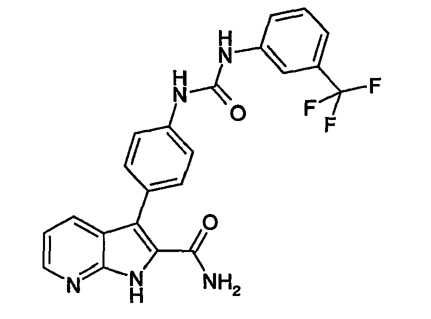

56.5 mg de solide blanc de 3-{4-[3-(3-Trifluorométhyl-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 3-Trifluorométhyl-phénylisocyanate.
Point de fusion : 263.6°C (Büchi)
Spectre de masse (ES⁺) : [M+H]⁺ = 440
Temps de rétention (min) : 3.95

### Exemple 21 : 3-{4-[3-(3,4-Diméthyl-phényl)-uréido]-phényl}-1H-pyrrolo[2,3-b]pyridine-2-carboxamide

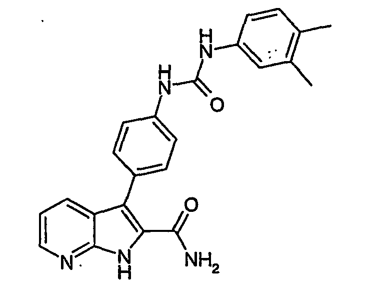

45.2 mg de solide blanc de 3-{4-[3-(3,4-Diméthyl-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 3,4-Diméthyl-phénylisocyanate.
Point de fusion : 274.7 °C (Büchi)
Spectre de masse (ES⁺) : [M + H]⁺ = 400
Temps de rétention (min) : 3.75

### Exemple 22: 3-{4-[3-(2-Méthoxy-5-Méthyl-phényl)-uréido]-phényl}-1H-pyrrolo[2,3-b]pyridine-2-carboxamide.

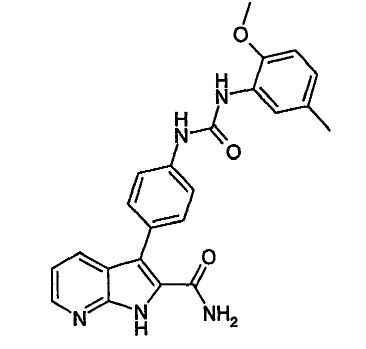

44.9 mg de solide beige de 3-{4-[3-(2-Méthoxy-5-Méthyl-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 2-Méthoxy-5-méthyl-phénylisocyanate.
Point de fusion: 327.7°C (Büchi)
Spectre de masse (ES⁺) : [M + H]⁺ = 416
Temps de rétention (min) : 3.76

### Exemple 23 : 3-[4-(3-m-Tolyl-uréido)-phényl]-1H-pyrrolo[2,3-b]pyridine-2-carboxamide

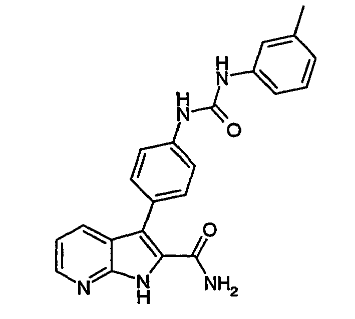

62.5 mg de solide beige de 3-[4-(3-m-Tolyl-uréido)-phényl]-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de m-tolylisocyanate.
Point de fusion : 266 °C (Büchi)
Spectre de masse (ES⁺) : [M+H]⁺ = 386
Temps de rétention (min) : 3.60

### Exemple 24 : 3-{4-[3-(4-Fluoro-phényl)-uréido]-phényl}-1H-pyrrolo[2,3-b]pyridine-2-carboxamide

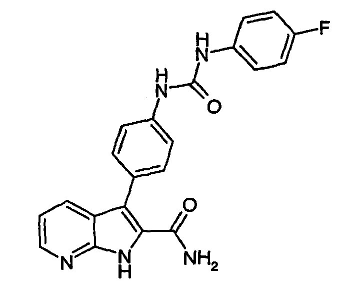

49.7 mg de solide beige de 3-{4-[3-(4-Fluoro-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 4-Fluoro-phénylisocyanate.
Point fusion : 299.9°C (Büchi)
Spectre de masse (ES⁺) : [M+H]⁺ = 390
Temps de rétention (min) : 3.45

### Exemple 25 : 3-[4-(3-p-Tolyl-uréido)-phényl]-1H-pyrrolo[2,3-b]pyridine-2-carboxamide

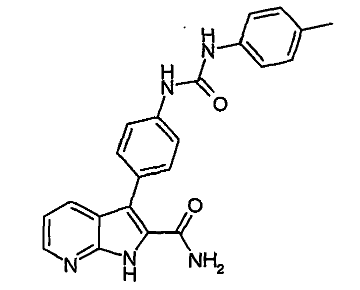

68.4 mg de solide beige de 3-[4-(3-p-Tolyl-uréido)-phényl]-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de p-tolylisocyanate.
Point de fusion : 293°C (Büchi)
Spectre de masse (ES⁺) : [M+H]⁺ = 386
Temps de rétention (min) : 3.58

### Exemple 26 : 3-{4-[3-(4-Méthyl-3-trifluorométhyl-phényl)-uréido]-phényl}-1H-pyrrolo[2,3-b]pyridine-2-carboxamide

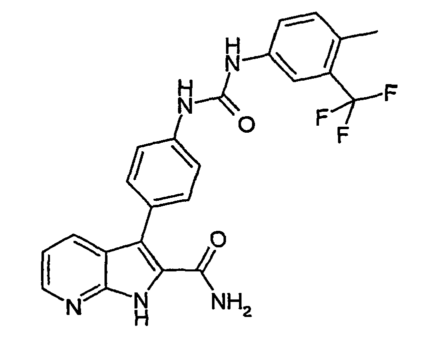

47.1 mg de solide blanc de 3-{4-[3-(4-Méthyl-3-trifluorométhyl-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 4-Méthyl-3-trifluorométhyl-phénylisocyanate.
Point de fusion: 285°C
Spectre de masse (ES⁺) : [M+H]⁺ = 454
Temps de rétention (min) : 4.10

### Exemple 27 : 3-{4-[3-(4-Difluorométhoxy-phényl)-uréido]-phényl}-1H-pyrrolo[2,3-b]pyridine-2-carboxamide

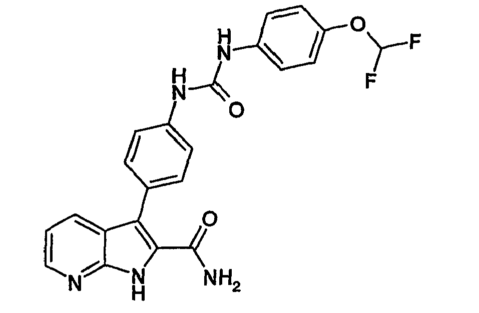

47.5 mg de solide blanc de 3-{4-[3-(4-Difluorométhoxy-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 4-Difluorométhoxy-phénylisocyanate.
Point de fusion : 283.5°C (Büchi)
Spectre de masse (ES⁺) : [M+H]⁺ = 438
Temps de rétention (min) : 3.64

### Exemple 28 : 3-{4-[3-(3,5-Diméthoxy-phényl)-uréido]-phényl}-1H-pyrrolo[2,3-b]pyridine-2-carboxamide

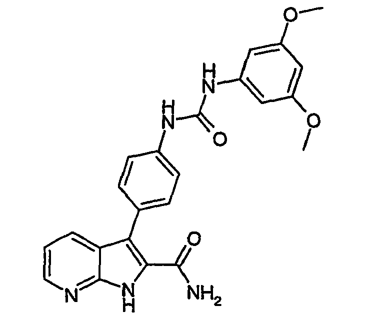

59.2 mg de solide beige de 3-{4-[3-(3,5-Diméthoxy-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 3,5-Diméthoxy-phénylisocyanate.
Point de fusion : 266.5°C (Büchi)
Spectre de masse (ES⁺) : [M+H]⁺ = 432
Temps de rétention (min) : 3.45

### Exemple 29 : 3-{4-[3-(4-Chloro-3-trifluorométhyl-phényl)-uréido]-phényl}-1H-pyrrolo[2,3-b]pyridine-2-carboxamide

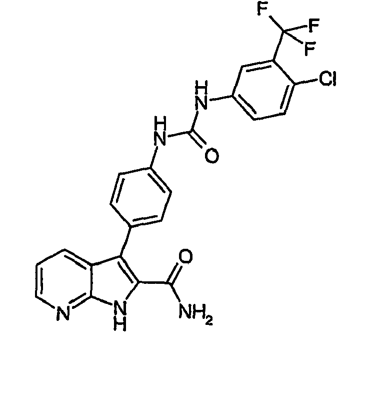

29.8 mg de solide blanc de 3-{4-[3-(4-Chloro-3-trifluorométhyl-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 4-Chloro-3-trifluorométhyl-phénylisocyanate.
Point de fusion: 311.1°C (Büchi)
Spectre de masse (ES⁺) : [M + H]⁺ = 474
Temps de rétention (min) : 4.22

### Exemple 30 : 3-{4-[3-(2,5-Diméthoxy-phényl)-uréido]-phényl}-1H-pyrrolo[2,3-b]pyridine-2-carboxamide

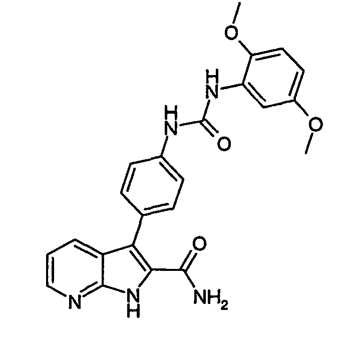

33.1 mg de lyophilisat jaune de 3-{4-[3-(2,5-Diméthoxy-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 2,5-Diméthoxy-phénylisocyanate.
Spectre de masse : LC-MS-DAD-ELSD :432 (+) = (M + H)(+) ; 430 (-) = (M - H)(-)
Temps de rétention (min) : 3.53

### Exemple 31 :3-{4-[3-(3-Fluoro-phényl)-uréido]-phényl}-1H-pyrrolo[2,3-b]pyridine-2-carboxamide.

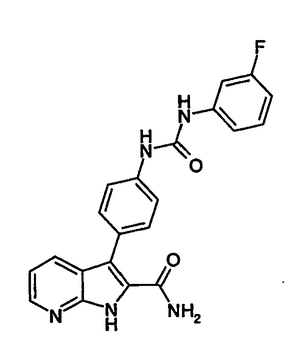

31.5 mg de lyophilisat blanc de 3-{4-[3-(3-Fluoro-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 3-Fluoro-phénylisocyanate.
Spectre de masse LC-MS-DAD-ELSD : 390(+) = (M+H)(+) ; 388(-) = (M-H)(-)
Temps de rétention (min) : 3.55

### Exemple 32:3-{4-[3-(2-Méthoxy-5-trifluorométhyl-phényl)-uréido]-phényl}-1H-pyrrolo[2,3-b]pyridine-2-carboxamide

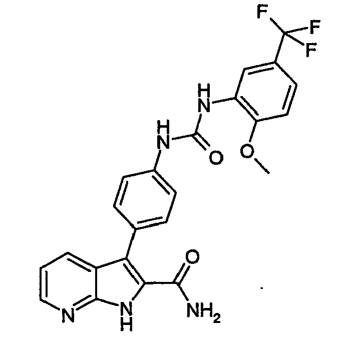

50 mg de solide beige de 3-{4-[3-(2-Méthoxy-5-trifluorométhyl-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 2-Méthoxy-5-trifluorométhyl-phénylisocyanate.
Point de fusion : 221 °C (Köfler-sublimation)
Spectre de masse LC-MS-DAD-ELSD : 470(+) = (M+H)(+) 468(-) = (M-H)(-)

### Exemple 33: Trifluoroacétate de 3-{4-[3-(2-Acetylamino-5-trifluorométhyl-phényl)-uréido]-phényl}-1H-pyrrolo[2,3-c]pyridine-2-carboxamide.

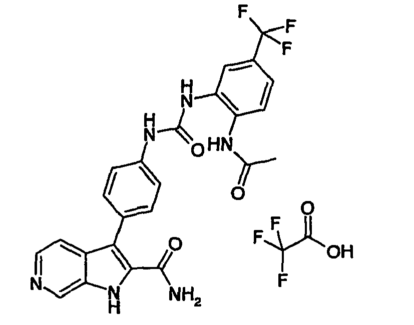

12 mg de solide jaune de trifluoroacétate de 3-{4-[3-(2-Acetylamino-5-trifluorométhyl-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 2-Acétylamino-5-trifluorométhyl-phénylisocyanate.
Spectre de masse (ES⁺) : [M + H]⁺ = 497
Temps de rétention (min) : 2.63

### Exemple 34:3-{4-[3-(2-Méthoxy-phényl)-uréido]-phényl}-1H-pyrrolo[2,3-c]pyridine-2-carboxamide

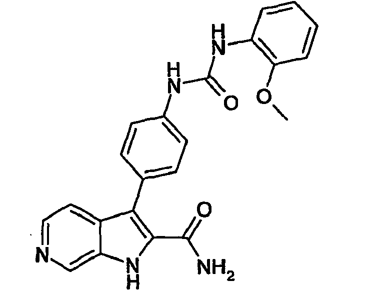

25 mg de solide jaune de 3-{4-[3-(2-Méthoxy-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 2-Méthoxy-phénylisocyanate.
Point de fusion : 216°C (Köfler)
Spectre de masse (ES⁺) : [M+H]⁺ = 402
Temps de rétention (min) : 3.06

### Exemple 35 : 3{4-[3-(2-Trifluorométhyl-phényl)-uréido]-phényl}-1H-pyrolo[2,3-c]pyridine-2-carboxamide.

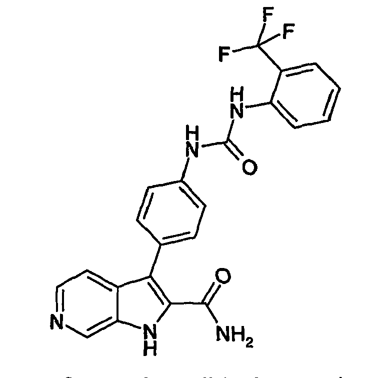

80 mg de solide jaune de 3-{4-[3-(2-Trifluorométhyl-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 2-Trifluorométhyl-phénylisocyanate.
Point de fusion : 228°C (Köfler)
Spectre de masse (ES⁺) : [M + H]⁺ = 440
Temps de rétention (min) : 3.17

### Exemple 36 : 3-{4-[3-(3-Trifluorométhyl-phényl)-uréido]-phényl}-1H-pyrrolo[2,3-c]pyridine-2-carboxamide

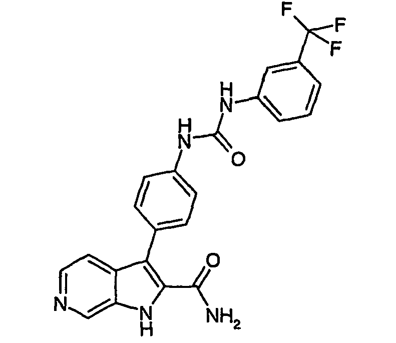

77 mg de solide jaune de 3-{4-[3-(3-Trifluorométhyl-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 3-Trifluorométhyl-phénylisocyanate.
Point de fusion : 256°C (Büchi B-545)
Spectre de masse (ES⁺) : [M+H]⁺ = 440
Temps de rétention (min) : 3.48

### Exemple 37 : 3-{4-[3-(4-Fluoro-phényl)-uréido]-phényl}-1H-pyrrolo[2,3-c]pyridine-2-carboxamide

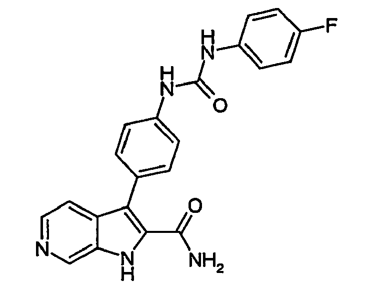

73 mg de solide jaune de 3-{4-[3-(4-Fluoro-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 4-Fluoro-phénylisocyanate.
Point de fusion : 271°C (Büchi B-545)
Spectre de masse (ES⁺) : [M+H]⁺ = 390
Temps de rétention (min) : 2.93

### Exemple 38 : 3-{4-[3-(4-Trifluorométhyl-phényl)-uréido]-phényl}-1H-pyrrolo[2,3-c]pyridine-2-carboxamide

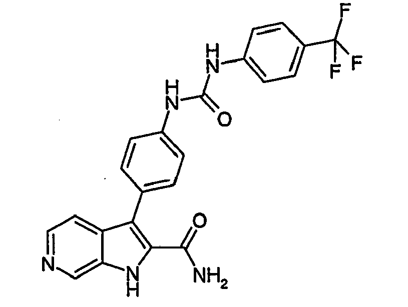

91 mg de solide jaune de 3-{4-[3-(4-Trifluorométhyl-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 4-Trifluorométhyl-phénylisocyanate.
Point de fusion : 289°C
Spectre de masse (ES⁺) : [M+H]⁺ = 440
Temps de rétention (min) : 3.48

### Exemple 39 : 3-[4-(3-p-Tolyl-uréido)-phényl]-1H-pyrrolo[2,3-c]pyridine-2-carboxamide

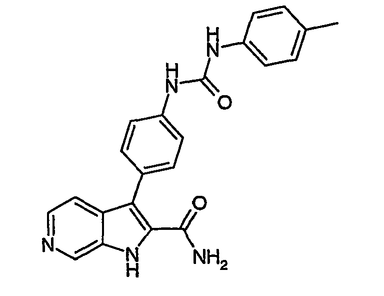

76 mg de solide jaune de 3-[4-(3-p-Tolyl-uréido)-phényl]-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de p-tolylisocyanate.
Point de fusion : 277°C (Büchi B-545)
Spectre de masse (ES⁺) : [M+H]⁺ = 386
Temps de rétention (min) : 3.13

### Exemple 40: 3-{4-[3-(4-Chloro-3-trifluorométhyl-phényl)-uréido]-phényl}-1H-pyrrolo[2,3-c]pyridine-2-carboxamide

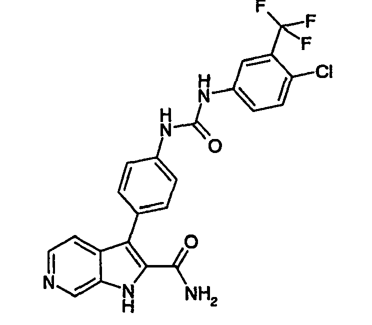

103 mg de solide jaune de 3-{4-[3-(4-Chloro-3-trifluorométhyl-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 4-Chloro-3-trifluorométhyl-phénylisocyanate.
Point de fusion: 228°C (Büchi B-545)
Spectre de masse (ES⁺) : [M + H]⁺ = 474
Temps de rétention (min) : 3.64

### Exemple 41 : 3-{4-[3-(2-Chloro-5-trifluorométhyl-phényl)-uréido]-phényl}-1H-pyrrolo[2,3-c]pyridine-2-carboxamide

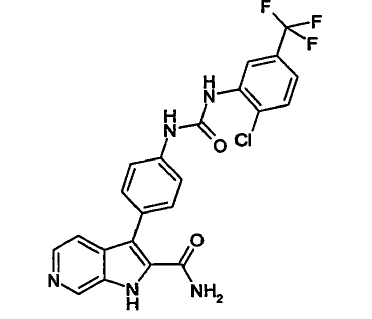

76 mg de solide jaune de 3-{4-[3-(2-Chloro-5-trifluorométhyl-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 2-Chloro-5-trifluorométhyl-phénylisocyanate.
Point de fusion: 243°C (Büchi B-545)
Spectre de masse (ES⁺) : [M+H]⁺ = 474
Temps de rétention (min) : 3.56

### Exemple 42: 3-{4-[3-(4-TrifluoroMéthoxy-phényl)-uréido]-phényl}-1H-pyrrolo[2,3-c]pyridine-2-carboxamide

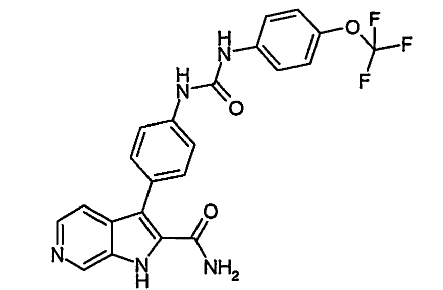

94 mg de solide jaune de 3-{4-[3-(4-TrifluoroMéthoxy-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 4-Trifluorométhoxy-phénylisocyanate.
Point de fusion : 276°C (Büchi B-545)
Spectre de masse (ES⁺) : [M+H]⁺ = 456
Temps de rétention (min) : 3.63

### Exemple 43: 3-{4-[3-(4-Difluorométhoxy-phényl)-uréido]-phényl}-1H-pyrrolo[2,3-c]pyridine-2-carboxamide

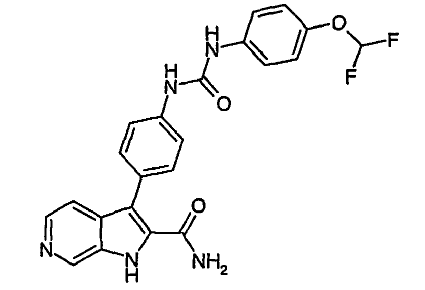

87 mg de solide jaune de 3-{4-[3-(4-Difluorométhoxy-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 4-Difluorométhoxy-phénylisocyanate.
Point de fusion : 257°C
Spectre de masse (ES⁺) : [M+H]⁺ =4.38
Temps de rétention (min) : 3.23

### Exemple 44 : 3-{4-[3-(3,4-Diméthyl-phényl)-uréido]-phényl}-1H-pyrrolo[2,3-c]pyridine-2-carboxamide

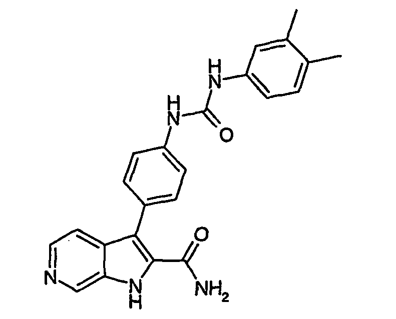

82 mg de solide jaune de 3-{4-[3-(3,4-Diméthyl-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 3,4-Diméthyl-phénylisocyanate.
Point de fusion : 230°C (Büchi B-545)
Spectre de masse (ES⁺) : [M+H]⁺ = 400
Temps de rétention (min) : 3.32

### Exemple 45 : 3-{4-[3-(3,5-Diméthoxy-phényl)-uréido]-phényl}-1H-pyrrolo[2,3-c]pyridine-2-carboxamide

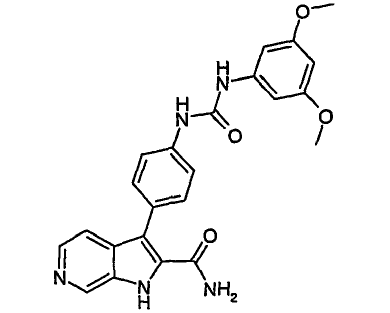

87 mg de solide jaune de 3-{4-[3-(3,5-Diméthoxy-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 3,5-Diméthoxy-phénylisocyanate.
Point de fusion : 225°C (Büchi B-545)
Spectre de masse (ES⁺) : [M+H]⁺ = 432
Temps de rétention (min) : 3.07

### Exemple 46: 3-{4-[3-(2,5-Diméthyl-phényl)-uréido]-phényl}-1H-pyrrolo[2,3-c]pyridine-2-carboxamide

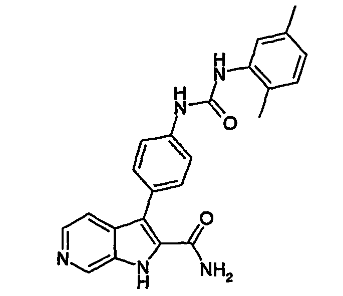

87 mg de solide jaune de 3-{4-[3-(2,5-Diméthyl-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 2,5-Diméthyl-phénylisocyanate.
Point de fusion : 261 °C (Büchi B-545)
Spectre de masse (ES⁺) : [M+H]⁺ = 400
Temps de rétention (min) : 3.25

### Exemple 47 :3-{4-[3-(2-Fluoro-phényl)-uréido]-phényl}-1H-pyrrolo[2,3-c]pyridine-2-carboxamide

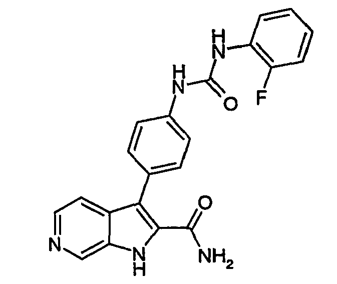

59 mg de solide jaune pâle de 3-{4-[3-(2-Fluoro-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 2-Fluoro-phénylisocyanate.
Point de fusion : 242°C (Büchi B-545)
Spectre de masse (ES⁺) : [M + H]⁺ = 390
Temps de rétention (min) : 2.41

### Exemple 48 : 3-{4-[3-(3-Fluoro-phényl)-uréido]-phényl}-1H-pyrrolo[2,3-c]pyridine-2-carboxamide

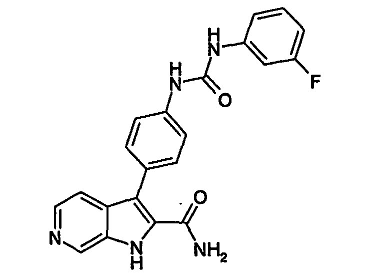

63 mg de solide jaune pâle de 3-{4-[3-(3-Fluoro-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 3-Fluoro-phénylisocyanate.
Point de fusion : 252°C (Büchi B-545)
Spectre de masse (ES+) : [M + H]+ = 390
Temps de rétention (min) : 2.55

### Exemple 49 : 3-{4-[3-(2-Fluoro-3-trifluorométhyl-phényl)-uréido]-phényl}-1 H-pyrrolo[2,3-c]pyridine-2-carboxamide

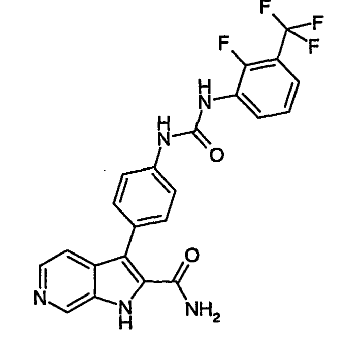

69 mg de solide jaune pâle de 3-{4-[3-(2-Fluoro-3-trifluorométhyl-phényl)-uréido]-phényl}-1H-pyrrolo[2,3-c]pyridine-2-carboxamide sont préparés comme décrit dans l'exemple 1 à partir de 3-(4-Aminophényl)-1 H-pyrrolo[2,3-c]pyridine-2-carboxamide et de 2-Fluoro-3-trifluorométhyl-phénylisocyanate.
Point de fusion: 240°C (Büchi B-545)
Spectre de masse (ES⁺) : [M + H]⁺ = 458
Temps de rétention (min) : 2.75

### Exemple 50 : 3-{4-[3-(3-Fluoro-5-trifluorométhyl-phényl)-uréido]-phényl}-1H-pyrrolo[2,3-c]pyridine-2-carboxamide

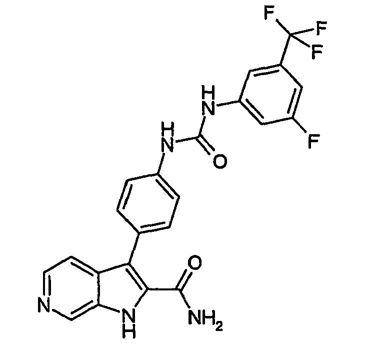

69 mg de solide jaune pâle de 3-{4-[3-(3-Fluoro-5-trifluorométhyl-phényl)-uréido]-phényl}-1H-pyrrolo[2,3-c]pyridine-2-carboxamide sont préparés comme décrit dans l'exemple 1 à partir de 3-(4-Aminophényl)-1 H-pyrrolo[2,3-c]pyridine-2-carboxamide et de 3-Fluoro-5-trifluorométhyl-phénylisocyanate.
Point de fusion: 261 °C (Büchi B-545)
Spectre de masse (ES⁺) : [M + H]⁺ = 458
Temps de rétention (min) : 2.88

### Exemple 51 : 3-{4-[3-(4-Fluoro-3-trifluorométhyl-phényl)-uréido]-phényl}-1H-pyrrolo[2,3-c]pyridine-2-carboxamide

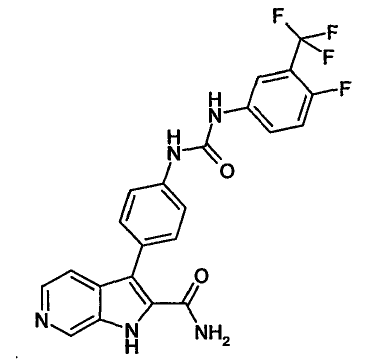

56 mg de solide jaune pâle de 3-{4-[3-(4-Fluoro-3-trifluorométhyl-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 4-Fluoro-3-trifluorométhyl-phénylisocyanate.
Point de fusion: 201°C (Büchi B-545)
Spectre de masse (ES⁺) : [M + H]⁺ = 458
Temps de rétention (min) : 2.85

### Exemple 52 : 3-{4-[3-(4-Méthyl-3-trifluorométhyl-phényl)-uréido]-phényl}-1H-pyrrolo[2,3-c]pyridine-2-carboxamide

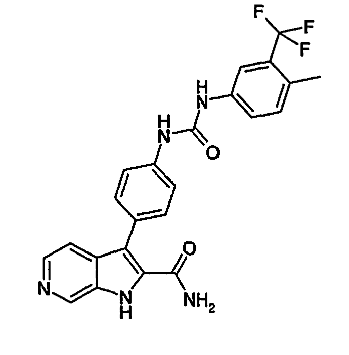

61 mg de solide jaune pâle de 3-{4-[3-(4-Méthyl-3-trifluorométhyl-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 4-Méthyl-3-trifluorométhyl-phénylisocyanate.
Point de fusion: 199°C
Spectre de masse (ES⁺) : [M + H]⁺ = 454
Temps de rétention (min) : 2.84

### Exemple 53: Trifluoroacétate de 3-{4-[3-(3-Méthoxy-phényl)-uréido]-phényl}-1H-pyrrolo[2,3-c]pyridine-2-carboxamide

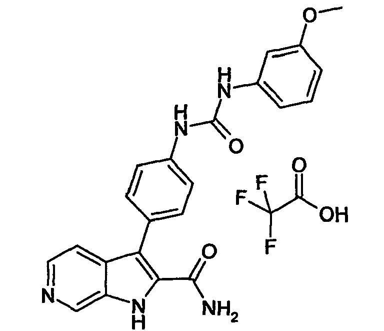

33.3 mg de lyophilisat jaune de trifluoroacétate de 3-{4-[3-(3-Méthoxy-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 3-Méthoxy-phénylisocyanate.
Spectre de masse (ES⁺) : [M + H]⁺ = 402
Temps de rétention (min) : 2.60

### Exemple 54 : Trifluoroacétate de 3-{4-[3-(3,4-Diméthoxy-phényl)-uréido]-phényl}-1H-pyrrolo[2,3-c]pyridine-2-carboxamide

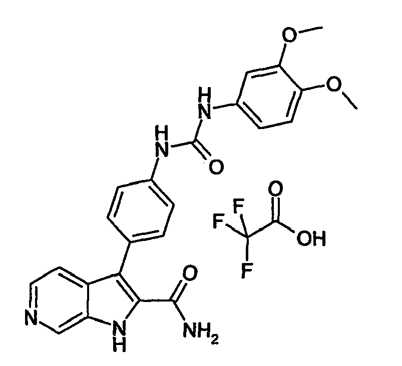

80.5 mg de lyophilisat jaune de trifluoroacétate de 3-{4-[3-(3,4-Diméthoxy-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 3,4-Diméthoxy-phénylisocyanate.
Spectre de masse (ES⁺) : [M + H]⁺ = 432
Temps de rétention (min) : 2.27

### Exemple 55 : Trifluoroacétate de 3-{4-[3-(2,5-Diméthoxy-phényl)-uréido]-phényl}-1H-pyrrolo[2,3-c]pyridine-2-carboxamide

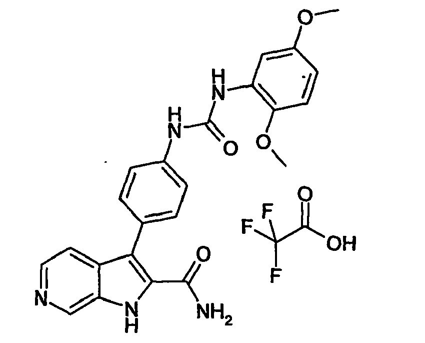

90.7 mg de lyophilisat jaune de trifluoroacétate de 3-{4-[3-(2,5-Diméthoxy-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 2,5-Diméthoxy-phénylisocyanate.
Spectre de masse (ES⁺) : [M + H]⁺= 432
Temps de rétention (min) : 2.62

### Exemple 56 : 3-[4-(3-o-Tolyl-uréido)-phényl]-1H-pyrrolo[2,3-c]pyridine-2-carboxamide

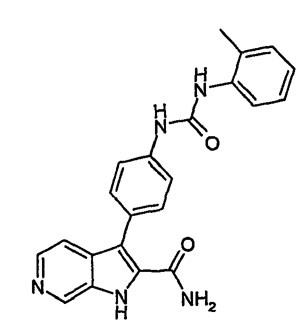

75.3 mg de solide jaune pâle de 3-[4-(3-o-Tolyl-uréido)-phényl]-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de o-Tolylisocyanate.
Point de fusion : 270°C (Büchi B-545)
Spectre de masse (ES⁺) : [M + H]⁺ = 386
Temps de rétention (min) : 2.54

### Exemple 57 :3-{4-[3-(4-Méthoxy-phényl)-uréido]-phényl}-1H-pyrrolo[2,3-c]pyridine-2-carboxamide

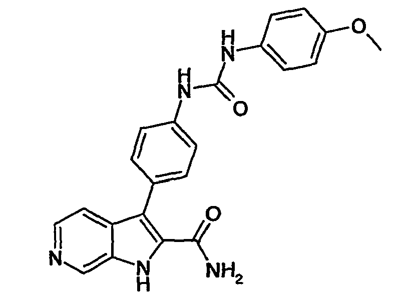

51.1 mg de solide jaune pâle de 3-{4-[3-(4-Méthoxy-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 4-Méthoxy-phénylisocyanate.
Point de fusion: 275°C (Büchi B-545)
Spectre de masse (ES+) : [M + H]+ = 402
Temps de rétention (min) : 2.28

### Exemple 58 : 3-{4-[3-(3-Chloro-4-Difluorométhoxy-phényl)-uréido]-phényl}-1H-pyrrolo[2,3-c] pyridine-2-carboxamide

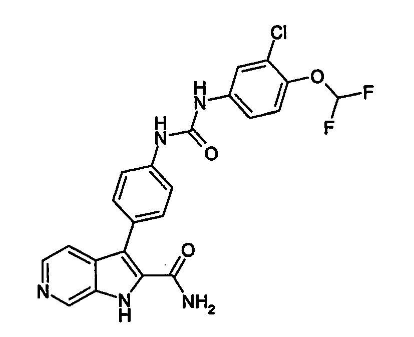

93 mg de solide jaune pâle de 3-{4-[3-(3-Chloro-4-Difluorométhoxy-phényl)-uréido]-phényl}-1H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 3-Chloro-4-Difluorométhoxy-phénylisocyanate.
Point de fusion : 267°C (Büchi B-545)
Spectre de masse (ES⁺) : [M + H]⁺= 472
Temps de rétention (min) : 2.90

### Exemple 59 : 3-{4-[3-(3,5-Diméthyl-phényl)-uréido]-phényl}-1H-pyrrolo[2,3-c]pyridine-2-carboxamide

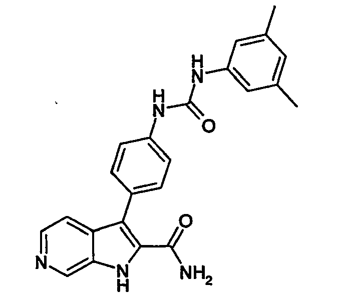

61 mg de solide jaune pâle de 3-{4-[3-(3,5-Diméthyl-phényl)-uréido]-phényl}-1 H-pyrrolo[2,3-c]pyridine-2-carboxamide sont préparés comme décrit dans l'exemple 1 à partir de 3-(4-Aminophényl)-1H-pyrrolo[2,3-c]pyridine-2-carboxamide et de 3,5-Diméthyl-phénylisocyanate.
Point de fusion : 188°C (Büchi B-545)
Spectre de masse (ES⁺) : [M + H]⁺ = 400
Temps de rétention (min) : 2.68

### Exemple 60 : 3-{4-[3-(3-éthyl-phényl)-uréido]-phényl}-1H-pyrrolo[2,3-c]pyridine-2-carboxamide

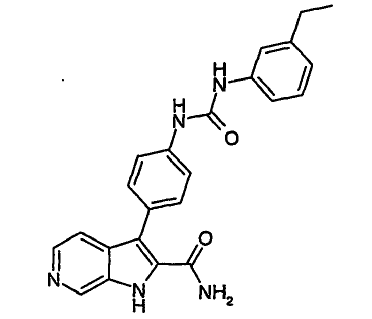

61 mg de solide jaune pâle de 3-{4-[3-(3-éthyl-phényl)-uréido]-phényl}-1H-pyrrolo[2,3-c]pyridine-2-carboxamidesont préparés comme décrit dans l'exemple 1 à partir de 3-(4-aminophényl)-1H-pyrrolo[2,3-c]pyridine-2-carboxamide et de 3-éthyl-phénylisocyanate.
Point de fusion : 257°C (Büchi B-545)
Spectre de masse (ES⁺) : [M + H]⁺ = 400
Temps de rétention (min) : 2.97

### Exemple 61 : 3-{4-[3-(3-éthyl-phényl)-uréido]-phényl}-1H-pyrrolo[2,3-b]pyridine-2-carboxamide

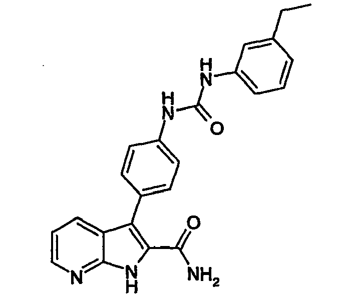

0.8 mg de solide blanc de 3-{4-[3-(3-éthyl-phényl)-uréido]-phényl}-1H-pyrrolo[2,3-b]pyridine-2-carboxamide sont préparés comme décrit dans l'exemple 7 à partir de 3-(4-Aminophényl)-1H-pyrrolo[2,3-b]pyridine-2-carboxamide et de 3-méthyl-phénylisocyanate.
Point de fusion : 254 °C (Büchi)
Spectre de masse (ES⁺) : [M+H]⁺= 400
Temps de rétention (min) : 7.18

### Détermination de l'activité des composés - Protocoles expérimentaux

### 1. FAK

L'activité inhibitrice des composés sur FAK est déterminée par une mesure de l'inhibition de l'autophosphorylation de l'enzyme en utilisant un test de fluorescence résolue dans le temps (HTRF).

L'ADNc complet de FAK humain, dont l'extrémité N-terminale a été marquée à l'histidine, a été cloné dans un vecteur d'expression baculovirus pFastBac HTc. La protéine a été exprimée et purifiée à environ 70% d'homogénéité.

L'activité kinase est déterminée en incubant l'enzyme (6.6 µg/ml) avec différentes concentrations de composé à tester dans un tampon 50 mM Hepes pH = 7,2, 10 mM MgCl₂, 100 µM Na₃VO₄ ,15 µM d'ATP pendant 1 heure à 37°C. La réaction enzymatique est stoppée par l'addition de tampon Hepes pH = 7,0 contenant 0.4 mM KF, 133 mM EDTA, 0.1 % BSA et le marquage est effectuée, pendant 1 à 2 heures à température ambiante, par l'addition dans ce tampon d'un anticorps anti-Histidine marqué avec XL665 et d'un anticorps monoclonal phosphospécifique de la tyrosine conjugué à du cryptate d'europium (Eu-K). Les caractéristiques des deux fluorophores sont disponibles dans G. Mathis et al., Anticancer Research, 1997, 17, pages 3011-3014. Le transfert d'énergie entre le cryptate d'europium excité vers le XL665 accepteur est proportionnel au degré d'autophosphorylation de FAK. Le signal de longue durée spécifique de XL-665 est mesuré dans un compteur de plaques Packard Discovery. Tous les essais sont effectués en double exemplaire et la moyenne des deux essais est calculée. L'inhibition de l'activité d'autophosphorylation de FAK avec des composés de l'invention est exprimée en pourcentage d'inhibition par rapport à un contrôle dont l'activité est mesurée en l'absence de composé test. Pour le calcul du % d'inhibition, le ratio [signal à 665 nm/signal à 620 nm] est considéré.

### 2. KDR

L'effet inhibiteur des composés est déterminé dans un test de phosphorylation de substrat par l'enzyme KDR *in vitro* par une technique de scintillation (plaque 96 puits, NEN).

Le domaine cytoplasmique de l'enzyme KDR humaine a été cloné sous forme de fusion GST dans le vecteur d'expression baculovirus pFastBac. La protéine a été exprimée dans les cellules SF21 et purifiée à environ 60 % d'homogénéité.

L'activité kinase de KDR est mesurée dans 20 mM MOPS, 10 mM MgCl2, 10 mM MnCl2, 1 mM DTT, 2.5 mM EGTA, 10 mM b-glycérophosphate, pH = 7.2, en présence de 10 mM MgCl₂, 100 µM Na₃VO₄, 1 mM NaF. 10 µl du composé sont ajoutés à 70 µl de tampon kinase contenant 100 ng d'enzyme KDR à 4°C. La réaction est lancée en ajoutant 20 µl de solution contenant 2 µg de substrat (fragment SH2-SH3 de la PLC□ exprimée sous forme de protéine de fusion GST), 2 µCi □ ³³P[ATP] et 2 µM ATP froid. Après 1 heure d'incubation à 37°C, la réaction est stoppée en ajoutant 1 volume (100 µl) de 200 mM EDTA. Le tampon d'incubation est retiré, et les puits sont lavés trois fois avec 300 µl de PBS. La radioactivité est mesurée dans chaque puits en utilisant un compteur de radioactivité Top Count NXT (Packard).

Le bruit de fond est déterminé par la mesure de la radioactivité dans quatre puits différents contenant l'ATP radioactif et le substrat seul.

Un contrôle d'activité totale est mesuré dans quatre puits différents contenant tous les réactifs (γ³³P-[ATP], KDR et substrat PLCγ) mais en l'absence de composé.

L'inhibition de l'activité KDR avec le composé de l'invention est exprimée en pourcentage d'inhibition de l'activité contrôle déterminée en l'absence de composé.

Le composé SU5614 (Calbiochem) (1 µM) est inclus dans chaque plaque comme contrôle d'inhibition.

### 3. Tie2

La séquence codante de Tie2 humain correspondant aux acides aminés du domaine intracellulaire 776-1124 a été générée par PCR en utilisant le cDNA isolé de placenta humain comme modèle. Cette séquence a été introduite dans un vecteur d'expression *baculovirus* pFastBacGT sous forme de protéine de fusion GST.

L'effet inhibiteur des molécules est déterminé dans un test de phosphorylation de PLC par Tie2 en présence de GST-Tie2 purifiée à environ 80% d'homogénéité. Le substrat est composé des fragments SH2-SH3 de la PLC exprimée sous forme de protéine de fusion GST.

L'activité kinase de Tie2 est mesurée dans un tampon MOPS 20mM pH 7.2, contenant 10 mM MgCl₂, 10 mM MnCl₂, 1 mM DTT, 10 mM de glycérophosphate. Dans une plaque 96 puits FlashPlate maintenue sur glace, on dépose un mélange réactionnel composé de 70 µL de tampon kinase contenant 100 ng d'enzyme GST-Tie2 par puits. Ensuite 10 µL de la molécule à tester diluée dans du DMSO à une concentration de 10 % maximum sont ajoutés. Pour une concentration donnée, chaque mesure est effectuée en quatre exemplaires. La réaction est initiée en ajoutant 20 µl de solution contenant 2 µg de GST-PLC, 2 µM d'ATP froid et 1 µCi d'³³P[ATP]. Après 1 heure d'incubation à 37°C, la réaction est stoppée en ajoutant 1 volume (100µl) d'EDTA à 200 mM. Après élimination du tampon d'incubation, les puits sont lavés trois fois avec 300 µL de PBS. La radioactivité est mesurée sur un MicroBeta1450 Wallac.

L'inhibition de l'activité Tie2 est calculée et exprimée en pourcentage d'inhibition par rapport à l'activité contrôle déterminée en l'absence de composé.

### 4. Aurora1 et Aurora2

L'effet inhibiteur de composés vis-à-vis des kinases Aurora1 et Aurora2 est déterminé par un test enzymatique utilisant une détection de radioactivité.

L'activité kinase de Aurora 1 et Aurora 2 est évaluée par la phosphorylation du substrat Numa-histidine en présence d'ATP radiomarqué ([³³P]ATP) en utilisant des plaques 96 puits Flashplate où le nickel-chelate est fixé à la surface de la microplaque. La quantité de phosphate ³³P incorporé au substrat NuMA est proportionnelle à l'activité de l'enzyme Aurora1 ou Aurora2.

### Protéines :

Les protéines sont produites dans le laboratoire de production de protéines du groupe Sanofi-Aventis.

Aurora 1 : complexe recombinant Aurora-B/INCENP-C3, purifié à environ 50% dont l'extrémité N-terminale de Aurora-B a été marquée à l'histidine.

Aurora 2 : protéine recombinante entière comprenant une queue histidine en N-terminal, a été exprimée dans E.coli et purifiée à plus de 82 %.

NuMA (protéine Nucléaire qui s'associe avec l'appareil mitotique) : fragment de 424 acides amines, exprimé dans E.coli dont l'extrémité N-terminale a été marquée à l'histidine et utilisé comme substrat pour les deux enzymes Aurora.

### Protocole :

Les microplaques utilisées sont des plaques Flash-Plate, 96 puits, nickel chélate (Perkin Elmer, modèle SMP107).

Les produits à évaluer sont incubés dans un volume réactionnel de 100 µL par puits, en présence de 10 nM de Aurora 1 ou Aurora 2, 500 nM de substrat NuMA dans un tampon composé de 50 mM de Tris/HCl (pH 7.5), 50 mM NaCl, 5 mM MgCl2 (Aurora-B) ou 10 mM MgCl2 (Aurora-A) et 1 mM de DTT, à 37°C.

Dans chaque puits, 80 µL du tampon d'incubation enzyme/substrat sont distribués puis 10 µL du produit à évaluer, en concentrations variables. La réaction est initiée par addition de 1µM d'ATP final contenant 0.2 µCi de [³³P]ATP (10 µL). Après 30 minutes d'incubation, la réaction est arrêtée par simple élimination du tampon réactionnel et chaque puits est lavé deux fois avec 300 µl du tampon Tris/HCl. La radioactivité est alors mesurée dans chaque puits à l'aide d'un appareil à scintillation, modèle Packard, Top count.

L'activité enzymatique contrôle d'Aurora est exprimée par le nombre de coup par minute obtenu en 30 minutes après déduction du bruit de fond ( mélange réactionnel ne contenant pas l'enzyme). L'évaluation des divers produits testés est exprimée en pourcentage d'inhibition de l'activité Aurora par rapport au contrôle.

### 5. CDK2/cycline E :

### Purification du complexe CDK2/CyclineE-(His)₆ par IMAC (Immobilized Metal Affinity Chromatography) :

Deux baculovirus recombinants portant les séquences humaines codant respectivement pour CDK2 et la CyclineE (cette dernière comportant un tag hexa-histidine en C terminal) sont utilisés pour co-infecter des cellules d'insecte Sf21. Deux à trois jours après le début de la co-infection, les cellules sont récoltées par centrifugation, puis conservées à -40°C jusqu'à leur utilisation. Après décongélation et lyse mécanique des cellules, le complexe présent dans le surnageant de lyse est purifié par chromatographie d'affinité sur Nickel (IMAC), et conservé à -80°C.

### Essai Flashplate CDK2/CyclinE en format 96 puits.

Un format en plaques 96 puits coatés à la streptavidine est utilisé pour tester l'activité des composés sur l'activité kinase de CDK2/Cycline E.

Pour réaliser cet essai, le substrat peptidique biotynilé, fragment de la protéine pRb, (biotinyl-SACPLNLPLQNNHTAADMYLSPVRSPKKKGSTTR-OH) est solubilisé à la concentration de 1 mM dans du tampon kinase (HEPES/ NaOH 50 mM, NaCl 1 mM, MgCl₂ 5 mM, pH 7.5) afin de constituer une solution-stock conservée à -20°C sous forme d'aliquotes de 110 µL. Le jour de l'expérience, un aliquote de cette solution est décongelé et dilué dans du tampon kinase contenant 1 mM de Dithiothréitol, ajouté au tampon extemporanément, afin d'obtenir une concentration de 14.3 µM. 70 µL de cette solution sont ajoutés dans chaque puits de la Flashplate afin d'obtenir une concentration finale en substrat de 10 µM lors de la réaction enzymatique conduite dans un volume final du milieu réactionnel de 100 µL (cf. ci-après).

Des dilutions intermédiaires d'inhibiteurs (produits de l'invention) à différentes concentrations sont préparéses dans le DMSO à partir de solutions stock à 10 mM dans des tubes séparés. On réalise ainsi des dilutions à 1000 µM, 333.3 µM, 111.1 µM, 37.03 µM, 12.35 µM, 4.11 µM et 1.37 µM. Un µL de chacune de ces solutions (ou 1 µL de DMSO pour les contrôles) est transféré dans les puits de la plaque de test.

Dans chaque puits, sont ensuite ajoutés 19 µl d'une solution d'un mélange d'adénosinetriphosphate (ATP) et d'ATPy³³P dans le tampon kinase à la concentration de 5,26 µM d'ATP total et de 52,6 µCi/ml de ³³P. La réaction enzymatique est déclenchée par addition de 10 µL par puits d'une solution de CDK2/Cycline E à 200 nM dans le tampon kinase contenant 1 mM de dithiothréitol (ou 10µL de tampon kinase contenant 1 mM de dithiothréitol pour les blancs réactionnels).

Après addition de chacun des réactifs, le volume final de chaque puits est de 100µL, la concentration finale de substrat est de 10 µM, les concentrations finales en inhibiteurs sont de10 µM, 3,33 µM, 1,11 µM, 0,37 µM, 0,123 µM, 0,041 µM et 0,014 µM (selon la concentration de la dilution intermédiaire), la concentration finale en ATP est de 1 µM, la quantité finale de ³³P est de 1µCi/puits, la concentration finale de complexe CDK2/Cycline E est de 20 nM.

Après l'addition de tous les réactifs, la plaque de test incubée à 30°C sous agitation orbitale à 650 rpm.

Lorsque l'incubation est terminée, la plaque est lavée trois fois par 300 µL par puits de PBS (Phosphate Buffered Saline, pH=7,4 sans calcium ni magnésium, référence 10010-015, Gibco BRL). L'incorporation de ³³P au peptide est quantifiée par comptage par scintillation avec un appareil Packard Topcount.NXT. L'activité inhibitrice des produits de l'invention est évaluée par mesure de la concentration d'inhibiteur permettant une diminution de l'activité enzymatique de 50 % (CI50).

### Résultats :

**Tableau 1 :**

| **Exemple** | **IC 50 (nM)** | | | | | |
|---|---|---|---|---|---|---|
| | **FAK** | **KDR** | **TIE2** | **Aurora A** | **Aurora B** | **CDK2** |
| **1** | 164 | 29 | 4 | 172 | 138 | |
| **2** | 299 | 150 | 21 | 222 | 196 | |
| **5** | 249 | 258 | 47 | 131 | 67 | |
| **7** | 184 | 34 | 9 | 553 | 133 | |

## Revendications

1. Produit répondant à la formule (I) suivante : 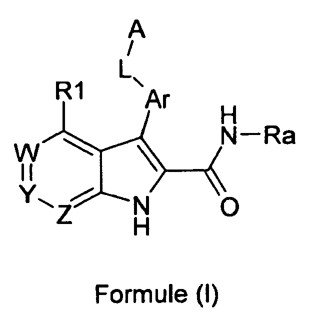 dans laquelle :
1) A et Ar sont des groupes phényle substitués;
2) L est NH-CO-NH;
3) W est C(R6), l'un de Y et Z, est choisi parmi N et NO, et l'autre est C(R5) et ;
4) R1, R5, et R6 sont H ;
5) Ra est H.

2. Produit selon la revendication 1, dans laquelle Y est N.

3. Produit selon la revendication 3, dans laquelle Z est N.

4. Produit selon la revendication 3, dans laquelle Z est NO.

5. Produit selon la revendication 1, dans laquelle A est substitué par un premier substituant sélectionné dans le groupe constitué par (C1-C12)alkyle, (C1-C3)alkyte halogéné, cyclo(C1-C3)alkyle, (C2-C12)alkylène, (C2-C12)alkynyle, (C6-C14)aryle, (C1-C13)hétéroaryle avec 1 à 4 hétéroatomes, O-(C1-C3)alkyle, O-(C1-C13)Aryle, O-(C1-C13)hétéroaryle, S-(C1-C3)alkyle, S-(C1-C13)Aryle, S-(C1-C13)hétéroaryle, chacun étant éventuellement substitué par un substituant choisi parmi (C1-C12)alkyle, halogène, O-(C1-C3)alkyle, N(R8)(R9) ; dans lequel R8 et R9 sont indépendamment choisis parmi H, (C1-C3)alkyle, (C1-C3)alkyleOH, (C1-C3)alkyle halogéné, (C1-C3)alkyleNH₂, (C1-C3)alkyleCOOM, (C1-C3)alkyleSO₃M; dans lequel lorsque R8 et R9 sont simultanément différents de H, ils peuvent être liés pour former un cycle de 5 à 7 chaînons comportant de 0 à 3 hétéroatomes choisis parmi O, N et S, et dans lequel M est H ou un cation de métal alcalin choisi parmi Li, Na et K.

6. Produit selon l'une quelconque des revendications 1 à 5, dans laquelle A est substitué par un deuxième substituant sélectionné dans le groupe constitué par F, Cl, Br, I, OH, SH, SO₃M. COOM, CN, NO₂, CON(R8)(R9), N(R8)CO(R9), (C1-C3)alkyle-OH, (C1-C3)alkyle-N(R8)(R9), (C1-C3)alkyle-(R10), (C1-C3)alkyle-COOH, N(R8)(R9); dans lequel R8 et R9 sont indépendamment choisis parmi H, (C1-C3)alkyle, (C1-C3)alkyleOH, (C1-C3)alkyle halogéné, (C1-C3)alkyleNH₂, (C1-C3)alkyleCOOM, (C1-C3)alkyleSO₃M ; dans lequel lorsque R8 et R9 sont simultanément différents de H, ils peuvent être liés pour former un cycle de 5 à 7 chaînons comportant de 0 à 3 hétéroatomes choisis parmi O, N et S; dans lequel M est H ou un cation de métal alcalin choisi parmi Li, Na et K ; et dans lequel R10 est H ou un hétérocycle non aromatique éventuellement substitué, comprenant 2 à 7 atomes de carbone, et 1 à 3 hétéroatomes choisis parmi N, O et S.

7. Produit selon l'une quelconque des revendications 5 à 6, dans laquelle A est phényle substitué par au moins un groupe choisi parmi halogène, (C1-C4)alkyle, (C1-C3)alkyle halogéné, O-(C1-C3)alkyle, S-(C1-C3)alkyle, O-(C1-C3)alkyle halogéné, S-(C1-C3)alkyle halogéné, et dans laquelle lorsque A est disubstitué, les deux substituants peuvent être liés entre eux pour former un cycle de 5 à 7 chaînons contenant de 0 à 3 hétéroatomes choisis parmi N, O et S.

8. Produit selon l'une quelconque des revendications précédentes, **caractérisé en ce qu'**il est sous forme :
non chirale, ou
racémique, ou
enrichie en un stéréo-isomère, ou
enrichie en un énantiomère ;
et **en ce qu'**il est éventuellement salifié.

9. Produit selon l'une quelconque des revendications précédentes, **caractérisé en ce qu'**il s'agit de :
3-{4-[3-(2-Fluoro-5-trifluorométhyl-phényl)-uréido]-phényl}-1H-pyrrolo[2,3-b]pyridine-2-carboxamide,
3-{4-[3-(2-Fluoro-phényl)-uréido]-phényl}-1H-pyrrolo[2,3-b]pyridine-2-carboxamide,
3-{4-[3-(2-Méthoxy-phényl)-uréido]-phényl}-1H-pyrrolo[2,3-b]pyridine-2-carboxamide,
3-{4-[3-(4-Trifluorométhyl-phényl)-uréido]-phényl}-1H-pyrrolo[2,3-b]pyridine-2-carboxamide,
3-{4-[3-(2-Chloro-5-trifluorométhyl-phényl)-uréido]-phényl}-1H-pyrrolo[2,3-b]pyridine-2-carboxamide,
3-{4-[3-(2-Fluoro-3-trifluorométhyl-phényl)-uréido]-phényl}-1H-pyrrolo[2,3-b]pyridine-2-carboxamide,
3-{4-[3-(4-Fluoro-3-trifluorométhyl-phényl)-uréido]-phényl}-1H-pyrrolo[2,3-b)pyridine-2-carboxamide,
3-{4-[3-(3-Fluoro-5-trifluorométhyl-phényl)-uréido]-phényl}-1H-pyrrolo[2,3-b]pyridine-2-carboxamide,
3-{4-[3-(4-TrifluoroMéthoxy-phényl)-uréido]-phényl}-1H-pyrrolo[2,3-b]pyridine-2-carboxamide,
3-{4-[3-(3,4-Diméthoxy-phényl)-uréido]-phényl}-1H-pyrrolo[2,3-b]pyridine-2-carboxamide,
3-{4-[3-(2,5-Diméthyl-phényl)-uréido]-phényl}-1H-pyrrolo[2,3-b]pyridine-2-carboxamide,
3-{4-[3-(3-Méthoxy-phényl)-uréido]-phényl}-1H-pyrrolo[2,3-b]pyridine-2-carboxamide,
3-{4-[3-(3-Trifluorométhyl-phényl)-uréido]-phényl}-1H-pyrrolo[2,3-b]pyridine-2-carboxamide,
3-{4-[3-(3,4-Diméthyl-phényl)-uréido]-phényl}-1H-pyrrolo[2,3-b]pyridine-2-carboxamide
3-{4-[3-(2-Méthoxy-5-Méthyl-phényl)-uréido]-phényl}-1H-pyrrolo[2,3-b]pyridine-2-carboxamide,
3-[4-(3-m-Tolyl-uréido)-phényl]-1H-pyrrolo[2,3-b]pyridine-2-carboxamide,
3-{4-[3-(4-Fluoro-phényl)-uréido]-phényl}-1H-pyrrolo[2,3-b]pyridine-2-carboxamide,
3-[4-(3-p-Tolyl-uréido)-phényl]-1H-pyrrolo[2,3-b]pyridine-2-carboxamide,
3-{4-[3-(4-Méthyl-3-trifluorométhyl-phényl)-uréido]-phényl}-1H-pyrrolo[2,3-b]pyridine-2-carboxamide,
3-{4-[3-(4-Difluorométhoxy-phényl)-uréido]-phényl}-1H-pyrrolo[2,3-b]pyridine-2-carboxamide
3-{4-[3-(3,5-Diméthoxy-phényl)-uréido]-phényl}-1H-pyrrolo[2,3-b]pyridine-2-carboxamide,
3-{4-[3-(4-Chloro-3-trifluorométhyl-phényl)-uréido]-phényl}-1H-pyrrolo[2,3-b]pyridine-2-carboxamide,
3-{4-[3-(2,5-Diméthoxy-phényl)-uréido]-phényl}-1H-pyrrolo[2,3-b]pyridine-2-carboxamide,
3-{4-[3-(3-Fluoro-phényl)-uréido]-phényl}-1H-pyrrolo[2,3-b]pyridine-2-carboxamide,
-3-{4-[3-(2-Méthoxy-5-trifluorométhyl-phényl)-uréido]-phényl}-1H-pyrrolo[2,3-b]pyridine-2-carboxamide,
3-{4-[3-(3-éthyl-phényl)-uréido]-phényl}-1H-pyrrolo[2,3-b]pyridine-2-carboxamide.

10. Produit selon l'une quelconque des revendications précédentes, **caractérisé en ce qu'**il s'agit de :
3-{4-[3-(2-Fluoro-5-trifluorométhyl-phényl)-uréido]-phényl}-7-oxy-1H-pyrrolo[2,3-b]pyridine-2-carboxamide.

11. Produit selon l'une quelconque des revendications précédentes, **caractérisé en ce qu'**il s'agit de :
3-{4-[3-(2-Fluoro-5-trifluorométhyl-phényl)-uréido]-phényl}-1H-pyrrolo[2,3-c]pyridine-2-carboxamide,
3-{4-[3-(2-Méthoxy-5-Méthyl-phényl)-uréido]-phényl}-1H-pyrrolo[2,3-c]pyridine-2-carboxamide,
Trifluoroacétate de 3-{4-[3-(3-chloro-phényl)-uréido]-phényl}-1H-pyrrolo[2,3-c]pyridine-2-carboxamide,
Trifluoroacetate de 3-{4-[3-(3-chloro-4-fluoro-phényl)-uréido]-phényl}-1H-pyrrolo[2,3-c]pyridine-2-carboxamide,
Trifluoroacétate de 3-{4-[3-(2-fluoro-5-Méthyl-phényl)-uréido]-phényl}-1H-pyrrolo[2,3-c]pyridine-2-carboxamide,
Trifluoroacétate de 3-[4-(3-m-tolyl-uréido)-phényl]-1H-pyrrolo[2,3-c]pyridine-2-carboxamide,
Trifluoroacétate de 3-{4-[3-(2-Acetylamino-5-trifluorométhyl-phényl)-uréido]-phényl}-1H-pyrrolo[2,3-c]pyridine-2-carboxamide,
3-{4-[3-(2-Méthoxy-phényl)-uréido]-phényl}-1H-pyrrolo[2,3-c]pyridine-2-carboxamide,
3-{4-[3-(2-Trifluorométhyl-phényl)-uréido]-phényl}-1H-pyrrolo[2,3-c]pyridine-2-carboxamide,
3-{4-[3-(3-Trifluorométhyl-phényl)-uréido]-phényl}-1H-pyrrolo[2,3-c]pyridine-2-carboxamide
3-{4-[3-(4-Fluoro-phényl)-uréido]-phényl}-1H-pyrrolo[2,3-c]pyridine-2-carboxamide,
3-{4-[3-(4-Trifluorométhyl-phényl)-uréido]-phényl}-1H-pyrrolo[2,3-c]pyridine-2-carboxamide,
3-[4-(3-p-Tolyl-uréido)-phényl]-1H-pyrrolo[2,3-c]pyridine-2-carboxamide,
3-{4-[3-(4-Chloro-3-trifluorométhyl-phényl)-uréido]-phényl}-1H-pyrrolo[2,3-c]pyridine-2-carboxamide,
3-{4-[3-(2-Chloro-5-trifluorométhyl-phényl)-uréido]-phényl}-1H-pyrrolo[2,3-c]pyridine-2-carboxamide,
3-{4-[3-(4-TrifluoroMéthoxy-phényl)-uréido]-phényl}-1H-pyrrolo[2,3-c]pyridine-2-carboxamide,
3-{4-[3-(4-Difluorométhoxy-phényl)-uréido]-phényl}-1H-pyrrolo[2,3-c]pyridine-2-carboxamide,
3-{4-[3-(3,4-Diméthyl-phényl)-uréido]-phényl}-1H-pyrrolo[2,3-c]pyridine-2-carboxamide,
3-{4-[3-(3,5-Diméthoxy-phényl)-uréido]-phényl}-1H-pyrrolo[2,3-c]pyridine-2-carboxamide
3-{4-[3-(2,5-Diméthyl-phényl)-uréido]-phényl}-1H-pyrrolo[2,3-c]pyridine-2-carboxamide,
3-{4-[3-(2-Fluoro-phényl)-uréido]-phényl}-1H-pyrrolo[2,3-c]pyridine-2-carboxamide,
3-{4-[3-(3-Fluoro-phényl)-uréido]-phényl}-1H-pyrrolo[2,3-c]pyridine-2-carboxamide,
3-{4-[3-(2-Fluoro-3-trifluorométhyl-phényl)-uréido]-phényl}-1H-pyrrolo[2,3-c]pyridine-2-carboxamide,
3-{4-[3-(3-Fluoro-5-trifluorométhyl-phényl)-uréido]-phényl}-1H-pyrrolo[2,3-c]pyridine-2-carboxamide,
3-{4-[3-(4-Fluoro-3-trifluorométhyl-phényl)-uréido]-phényl}-1H-pyrrolo[2,3-c]pyridine-2-carboxamide,
3-{4-[3-(4-Méthyl-3-trifluorométhyl-phényl)-uréido]-phényl}-1H-pyrrolo[2,3-c]pyridine-2-carboxamide,
Trifluoroacétate de 3-{4-[3-(3-Méthoxy-phényl)-uréido]-phényl}-1H-pyrrolo[2,3-c]pyridine-2-carboxamide,
Trifluoroacétate de 3-{4-[3-(3,4-Diméthoxy-phényl)-uréido]-phényl}-1H-pyrrolo[2,3-c]pyridine-2-carboxamide,
Trifluoroacétate de 3-{4-[3-(2,5-Diméthoxy-phényl)-uréido]-phényl}-1H-pyrrolo[2,3-c]pyridine-2-carboxamide,
3-[4-(3-o-Tolyl-uréido)-phényl]-1H-pyrrolo[2,3-c]pyridine-2-carboxamide,
3-{4-[3-(4-Méthoxy-phényl)-uréido]-phényl}-1H-pyrrolo[2,3-c]pyridine-2-carboxamide,
3-{4-[3-(3-Chloro-4-Difluorométhoxy-phényl)-uréido]-phényl}-1H-pyrrolo[2,3-c]pyridine-2-carboxamide,
3-{4-[3-(3,5-Diméthyl-phényl)-uréido]-phényl}-1H-pyrrolo[2,3-c]pyridine-2-carboxamide,
3-{4-[3-(3-éthyl-phényl)-uréido]-phényl}-1H-pyrrolo[2,3-c]pyridine-2-carboxamide.

12. Médicament, **caractérisé en ce qu'**il comprend un produit de formule (I) selon l'une quelconque des revendication 1 à 11, ou un sel d'addition de ce composé à un acide pharmaceutiquement acceptable, ou encore un hydrate ou un solvate du produit de formule (I).

13. Composition pharmaceutique comprenant un produit selon l'une quelconque des revendications précédentes, en combinaison avec un excipient pharmaceutiquement acceptable.

14. Utilisation d'un produit selon l'une quelconque des revendications 1 à 11, comme agent inhibiteur d'une réaction catalysée par une kinase.

15. Utilisation d'un produit selon la revendication 14, dans laquelle la kinase est choisie parmi FAK, KDR, Tie2, Aurora A, Aurora B, et CDK2.

16. Utilisation selon la revendication 15, dans laquelle la kinase est choisie parmi KDR et Tie2.

17. Utilisation d'un produit selon l'une quelconque des revendications 1 à 11, pour la fabrication d'un médicament utile pour traiter un état pathologique.

18. Utilisation selon la revendication 17, **caractérisée en ce que** l'état pathologique est le cancer.

## Claims

1. Product corresponding to formula (I) below: 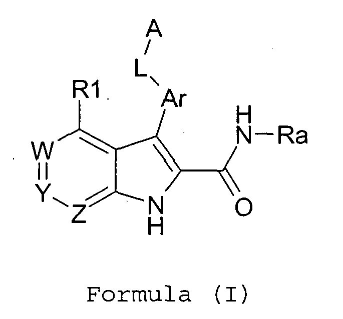 in which:
1) A and Ar are substituted phenyl groups;
2) L is NH-CO-NH;
3) W is C(R6), one from among Y and Z is chosen from N and NO, and the other is C(R5);
4) R1, R5, and R6 are H;
5) Ra is H.

2. Product according to Claim 1, in which Y is N.

3. Product according to Claim 1, in which Z is N.

4. Product according to Claim 1, in which Z is NO.

5. Product according to Claim 1, in which A is substituted with a first substituent selected from the group consisting of (C1-C12)alkyl, halo(C1-C3)alkyl, cyclo(C1-C3)alkyl, (C2-C12)alkylene, (C2-C12)alkynyl, (C6-C14)aryl, (C1-C13)heteroaryl with 1 to 4 heteroatoms, O-(C1-C3)alkyl, O-(C1-C13)aryl, O-(C1-C13)heteroaryl, S-(C1-C3)alkyl, S-(C1-C13)aryl, S-(C1-C13)heteroaryl, each being optionally substituted with a substituent chosen from (C1-C12)alkyl, halogen, O-(C1-C3)alkyl, N(R8)(R9); in which R8 and R9 are independently chosen from H, (C1-C3)alkyl, (C1-C3)alkylOH, halo(C1-C3)alkyl, (C1-C3)alkylNH₂, (C1-C3)alkylCOOM, (C1-C3)alkylSO₃M; in which, when R8 and R9 are simultaneously other than H, they may be bonded to form a 5- to 7-membered ring comprising from 0 to 3 heteroatoms chosen from O, N and S, and in which M is H or a cation of an alkali metal chosen from Li, Na and K.

6. Product according to any one of Claims 1 to 5, in which A is substituted with a second substituent selected from the group consisting of F, Cl, Br, I, OH, SH, SO₃M, COOM, CN, NO₂, CON(R8)(R9), N(R8)CO(R9), (C1-C3)alkyl-OH, (C1-C3)alkyl-N(R8)(R9), (C1-C3)alkyl-(R10), (C1-C3)alkyl-COOH, N(R8)(R9); in which R8 and R9 are independently chosen from H, (C1-C3)alkyl, (C1-C3)alkylOH, (C1-C3)haloalkyl, (C1-C3)alkylNH₂, (C1-C3)alkylCOOM, (C1-C3)alkylSO₃M; in which, when R8 and R9 are simultaneously other than H, they may be linked to form a 5- to 7-membered ring comprising from 0 to 3 heteroatoms chosen from O, N and S; in which M is H or a cation of an alkali metal chosen from Li, Na and K; and in which R10 is H or an optionally substituted non-aromatic heterocycle containing 2 to 7 carbon atoms and 1 to 3 heteroatoms chosen from N, O and S.

7. Product according to either of Claims 5 and 6, in which A is phenyl substituted with at least one group chosen from halogen, (C1-C4)alkyl, (C1-C3)haloalkyl, O-(C1-C3)alkyl, S-(C1-C3)alkyl, O-(C1-C3)haloalkyl, S-(C1-C3)haloalkyl, and in which, when A is disubstituted, the two substituents may be linked together to form a 5-to 7-membered ring containing from 0 to 3 heteroatoms chosen from N, O and S.

8. Product according to any one of the preceding claims, **characterized in that** it is:
in non-chiral form, or
in racemic form, or
enriched in one stereoisomer, or
enriched in one enantiomer;
and **in that** it is optionally salified.

9. Product according to any one of the preceding claims, **characterized in that** it is:
3-{4-[3-(2-fluoro-5-trifluoromethylphenyl)ureido]phenyl}-1H-pyrrolo[2,3-b]pyridine-2-carboxamide,
3-{4-[3-(2-fluorophenyl)ureido]phenyl}-1H-pyrrolo[2,3-b]pyridine-2-carboxamide,
3-{4-[3-(2-methoxyphenyl)ureido]phenyl}-1H-pyrrolo[2,3-b]pyridine-2-carboxamide,
3-{4-[3-(4-trifluoromethylphenyl)ureido]phenyl}-1H-pyrrolo[2,3-b]pyridine-2-carboxamide,
3-{4-[3-(2-chloro-5-trifluoromethylphenyl)ureido]phenyl}-1H-pyrrolo[2,3-b]pyridine-2-carboxamide,
3-{4-[3-(2-fluoro-3-trifluoromethylphenyl)ureido]phenyl}-1H-pyrrolo[2,3-b]pyridine-2-carboxamide,
3-{4-[3-(4-fluoro-3--trifluoromethylphenyl)ureido]phenyl}-1H-pyrrolo[2,3-b]pyridine-2-carboxamide,
3-{4-[3-(3-fluoro-5-trifluoromethylphenyl)ureido]phenyl}-1H-pyrrolo[2,3-b]pyridine-2-carboxamide,
3-{4-[3-(4-trifluoromethoxyphenyl)ureido]phenyl}-1H-pyrrolo[2,3-b]pyridine-2-carboxamide,
3-{4-[3-(3,4-dimethoxyphenyl)ureido]phenyl}-1H-pyrrolo[2,3-b]pyridine-2-carboxamide,
3-{4-[3-(2,5-dimethylphenyl)ureido]phenyl}-1H-pyrrolo[2,3-b]pyridine-2-carboxamide,
3-{4-[3-(3-methoxyphenyl)ureido]phenyl}-1H-pyrrolo[2,3-b]pyridine-2-carboxamide,
3-{4-[3-(3-trifluoromethylphenyl)ureido]phenyl}-1H-pyrrolo[2,3-b]pyridine-2-carboxamide,
3-{4-[3-(3,4-dimethylphenyl)ureido]phenyl}-1H-pyrrolo[2,3-b]pyridine-2-carboxamide
3-{4-[3-(2-methoxy-5-methylphenyl)ureido]phenyl}-1H-pyrrolo[2,3-b]pyridine-2-carboxamide,
3-[4-(3-m-tolylureido)phenyl]-1H-pyrrolo[2,3-b]pyridine-2-carboxamide,
3-{4-[3-(4-fluorophenyl)ureido]phenyl}-1H-pyrrolo[2,3-b]pyridine-2-carboxamide,
3-[4-(3-p-tolylureido)phenyl]-1H-pyrrolo[2,3-b]pyridine-2-carboxamide,
3-{4-[3-(4-methyl-3-trifluoromethylphenyl)ureido]phenyl}-1H-pyrrolo[2,3-b]pyridine-2-carboxamide,
3-{4-[3-(4-difluoromethoxyphenyl)ureido]phenyl}-1H-pyrrolo[2,3-b]pyridine-2-carboxamide
3-{4-[3-(3,5-dimethoxyphenyl)ureido]phenyl}-1H-pyrrolo[2,3-b]pyridine-2-carboxamide,
3-{4-[3-(4-chloro-3-trifluoromethylphenyl)ureido]phenyl}-1H-pyrrolo[2,3-b]pyridine-2-carboxamide,
3-{4-[3-(2,5-dimethoxyphenyl)ureido]phenyl}-1H-pyrrolo[2,3-b]pyridine-2-carboxamide,
3-{4-[3-(3-fluorophenyl)ureido]phenyl}-1H-pyrrolo[2,3-b]pyridine-2-carboxamide,
3-{4-[3-(2-methoxy-5-trifluoromethylphenyl)ureido]phenyl}-1H-pyrrolo[2,3-b]pyridine-2-carboxamide,
3-{4-[3-(3-ethylphenyl)ureido]phenyl}-1H-pyrrolo[2,3-b]pyridine-2-carboxamide.

10. Product according to any one of the preceding claims, **characterized in that** it is:
3-{4-[3-(2-fluoro-5-trifluoromethylphenyl)ureido]phenyl}-7-oxy-1H-pyrrolo[2,3-b]pyridine-2-carboxamide.

11. Product according to any one of the preceding claims, **characterized in that** it is:
3-{4-[3-(2-fluoro-5-trifluoromethylphenyl)ureido]phenyl}-1H-pyrrolo[2,3-c]pyridine-2-carboxamide,
3-{4-[3-(2-methoxy-5-methylphenyl)ureido]phenyl}-1H-pyrrolo[2,3-c]pyridine-2-carboxamide,
3-{4-[3-(3-chlorophenyl)ureido]phenyl}-1H-pyrrolo[2,3-c]pyridine-2-carboxamide trifluoroacetate,
3-{4-[3-(3-chloro-4-fluorophenyl)ureido]phenyl}-1H-pyrrolo[2,3-c]pyridine-2-carboxamide trifluoroacetate,
3-{4-[3-(2-fluoro-5-methylphenyl)ureido]phenyl}-1H-pyrrolo[2,3-c]pyridine-2-carboxamide trifluoroacetate,
3-[4-(3-m-tolylureido)phenyl]-1H-pyrrolo[2,3-c]pyridine-2-carboxamide trifluoroacetate,
3-{4-[3-(2-acetylamino-5-trifluoromethylphenyl)ureido]phenyl}-1H-pyrrolo[2,3-c]pyridine-2-carboxamide trifluoroacetate,
3-{4-[3-(2-methoxyphenyl)ureido]phenyl}-1H-pyrrolo[2,3-c]pyridine-2-carboxamide,
3-{4-[3-(2-trifluoromethylphenyl)ureido]phenyl}-1H-pyrrolo[2,3-c]pyridine-2-carboxamide,
3-{4-[3-(3-trifluoromethylphenyl)ureido]phenyl}-1H-pyrrolo[2,3-c]pyridine-2-carboxamide
3-{4-[3-(4-fluorophenyl)ureido]phenyl}-1H-pyrrolo[2,3-c]pyridine-2-carboxamide,
3-{4-[3-(4-trifluoromethylphenyl)ureido]phenyl}-1H-pyrrolo[2,3-c]pyridine-2-carboxamide,
3-[4-(3-p-tolylureido)phenyl]-1H-pyrrolo[2,3-c]pyridine-2-carboxamide,
3-{4-[3-(4-chloro-3-trifluoromethylphenyl)ureido]phenyl}-1H-pyrrolo[2,3-c]pyridine-2-carboxamide,
3-{4-[3-(2-chloro-5-trifluoromethylphenyl)ureido]phenyl}-1H-pyrrolo[2,3-c]pyridine-2-carboxamide,
3-{4-[3-(4-trifluoromethoxyphenyl)ureido]phenyl}-1H-pyrrolo[2,3-c]pyridine-2-carboxamide,
3-{4-[3-(4-difluoromethoxyphenyl)ureido]phenyl}-1H-pyrrolo[2,3-c]pyridine-2-carboxamide,
3-{4-[3-(3,4-dimethylphenyl)ureido]phenyl}-1H-pyrrolo[2,3-c]pyridine-2-carboxamide,
3-{4-[3-(3,5-dimethoxyphenyl)ureido]phenyl}-1H-pyrrolo[2,3-c]pyridine-2-carboxamide
3-{4-[3-(2,5-dimethylphenyl)ureido]phenyl}-1H-pyrrolo[2,3-c]pyridine-2-carboxamide,
3-{4-[3-(2-fluorophenyl)ureido]phenyl}-1H-pyrrolo[2,3-c]pyridine-2-carboxamide,
3-{4-[3-(3-fluorophenyl)ureido]phenyl}-1H-pyrrolo[2,3-c]pyridine-2-carboxamide,
3-{4-[3-(2-fluoro-3-trifluoromethylphenyl)ureido]phenyl-}-1H-pyrrolo[2,3-c]pyridine-2-carboxamide,
3-{4-[3-(3-fluoro-5-trifluoromethylphenyl)ureido]phenyl}-1H-pyrrolo[2,3-c]pyridine-2-carboxamide,
3-{4-[3-(4-fluoro-3-trifluoromethylphenyl)ureido]phenyl}-1H-pyrrolo[2,3-c]pyridine-2-carboxamide,
3-{4-[3-(4-methyl-3-trifluoromethylphenyl)ureido]phenyl}-1H-pyrrolo[2,3-c]pyridine-2-carboxamide,
3-{4-[3-(3-methoxyphenyl)ureido]phenyl}-1H-pyrrolo[2,3-c]pyridine-2-carboxamide trifluoroacetate,
3-{4-[3-(3,4-dimethoxyphenyl)ureido]phenyl}-1H-pyrrolo[2,3-c]pyridine-2-carboxamide trifluoroacetate,
3-{4-[3-(2,5-dimethoxyphenyl)ureido]phenyl}-1H-pyrrolo[2,3-c]pyridine-2-carboxamide trifluoroacetate,
3-[4-(3-o-tolylureido)phenyl]-1H-pyrrolo[2,3-c]pyridine-2-carboxamide,
3-{4-[3-(4-methoxyphenyl)ureido]phenyl}-1H-pyrrolo[2,3-c]pyridine-2-carboxamide,
3-{4-[3-(3-chloro-4-difluoromethoxyphenyl)ureido]phenyl}-1H-pyrrolo[2,3-c]pyridine-2-carboxamide,
3-{4-[3-(3,5-dimethylphenyl)ureido]phenyl}-1H-pyrrolo[2,3-c]pyridine-2-carboxamide,
3-{4-[3-(3-ethylphenyl)ureido]phenyl}-1H-pyrrolo[2,3-c]pyridine-2-carboxamide.

12. Medicament, **characterized in that** it comprises a product of formula (I) according to any one of Claims 1 to 11, or an addition salt of this compound with a pharmaceutically acceptable acid, or alternatively a hydrate or a solvate of the product of formula (I).

13. Pharmaceutical composition comprising a product according to any one of the preceding claims, in combination with a pharmaceutically acceptable excipient.

14. Use of a product according to any one of Claims 1 to 11, as an agent for inhibiting a reaction catalysed by a kinase.

15. Use of a product according to Claim 14, in which the kinase is chosen from FAK, KDR, Tie2, Aurora A, Aurora B and CDK2.

16. Use according to Claim 15, in which the kinase is chosen from KDR and Tie2.

17. Use of the product according to any one of Claims 1 to 11, for the manufacture of a medicament that is useful for treating a pathological condition.

18. Use according to Claim 17, **characterized in that** the pathological condition is cancer.

## Patentansprüche

1. Produkt gemäß der folgenden Formel (I) 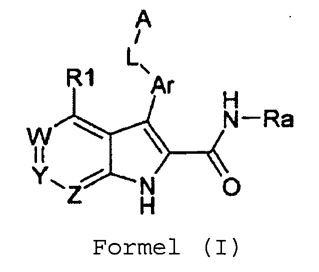 in der:
1) A und Ar substituierte Phenylgruppen bedeuten;
2) L NH-CO-NH bedeutet;
3) W C(R6) bedeutet, eines von Y und Z aus der Reihe N und NO stammt, während das andere C(R5) bedeutet;
4) R1, R5 und R6 H bedeuten;
5) Ra H bedeutet.

2. Produkt nach Anspruch 1, in dem Y N bedeutet.

3. Produkt nach Anspruch 1, in dem Z N bedeutet.

4. Produkt nach Anspruch 1, in dem Z NO bedeutet.

5. Produkt nach Anspruch 1, in dem A durch einen ersten Substituenten substituiert ist, der aus der Gruppe bestehend aus (C1-C12)-Alkyl, halogeniertem (C1-C3)-Alkyl, Cyclo(C1-C3)-alkyl, (C2-C12)-Alkylen, (C2-C12)-Alkinyl, (C6-C14)-Aryl, (C1-C13)-Heteroaryl mit 1 bis 4 Heteroatomen, O-(C1-C3)-Alkyl, O-(C1-C13)-Aryl, O-(C1-C13)-Heteroaryl, S-(C1-C3)-Alkyl, S-(C1-C13)-Aryl, S-(C1-C13)-Heteroaryl, von denen jeder gegebenenfalls durch einen Substituenten ausgewählt aus der Reihe (C1-C12)-Alkyl, Halogen, O-(C1-C3)-Alkyl, N(R8)(R9) substituiert ist, ausgewählt ist; in der R8 und R9 unabhängig aus der Reihe H, (C1-C3)-Alkyl, (C1-C3)-Alkyl-OH, halogeniertes (C1-C3)-Alkyl, (C1-C3)-Alkyl-NH₂, (C1-C3)-Alkyl-COOM, (C1-C3)-Alkyl-SO₃M ausgewählt sind; in der, wenn R8 und R9 gleichzeitig anders als H bedeuten, diese miteinander unter Bildung eines Zyklus mit 5 bis 7 Ringgliedern umfassend 0 bis 3 Heteroatome ausgewählt aus der Reihe O, N und S verbunden sein können, und in der M H oder ein Alkalimetallkation ausgewählt aus der Reihe Li, Na und K bedeutet.

6. Produkt nach einem der Ansprüche 1 bis 5, in dem A durch einen zweiten Substituenten ausgewählt aus der Gruppe bestehend aus F, Cl, Br, I, OH, SH, SO₃M, COOM, CN, NO₂, CON(R8) (R9), N(R8)CO(R9), (C1-C3)-Alkyl-OH, (C1-C3)-Alkyl-N(R8)(R9), (C1-C3)-Alkyl(R10), (C1-C3)-Alkyl-COOH, N(R8)(R9) substituiert ist; in der R8 und R9 unabhängig aus der Reihe H, (C1-C3)-Alkyl, (C1-C3)-Alkyl-OH, halogeniertes (C1-C3)-Alkyl, (C1-C3)-Alkyl-NH₂, (C1-C3)-Alkyl-COOM, (C1-C3)-Alkyl-SO₃M ausgewählt sind; in der, wenn R8 und R9 gleichzeitig anders als H bedeuten, diese miteinander unter Bildung eines Zyklus mit 5 bis 7 Ringgliedern umfassend 0 bis 3 Heteroatome ausgewählt aus der Reihe O, N und S verbunden sein können; in der M, H oder ein Alkalimetallkation ausgewählt aus der Reihe Li, Na und K bedeutet; und in der R10 H oder einen gegebenenfalls substituierten nichtaromatischen Heterocyclus umfassend 2 bis 7 Kohlenstoffatome und 1 bis 3 Heteroatome ausgewählt aus der Reihe N, O und S bedeutet.

7. Produkt nach einem der Ansprüche 5 bis 6, in dem A Phenyl, das durch mindestens eine Gruppe ausgewählt aus der Reihe Halogen, (C1-C4)-Alkyl, halogeniertes (C1-C3)-Alkyl, O-(C1-C3)-Alkyl, S-(C1-C3)-Alkyl, halogeniertes O-(C1-C3)-Alkyl, halogeniertes S-(C1-C3)-Alkyl substituiert ist, bedeutet, und in dem, wenn A disubstituiert ist, die beiden Substituenten miteinander unter Bildung eines Cyclus mit 5 bis 7 Ringgliedern, der 0 bis 3 Heteroatome ausgewählt aus der Reihe N, O und S enthält, verbunden sein können.

8. Produkt nach einem der vorhergehenden Ansprüche, **dadurch gekennzeichnet, daß** es in
nichtchiraler Form oder
in racemischer Form oder
in stereoisomerenangereicherter Form oder
in enantiomerenangereicherter Form
vorliegt und **dadurch**, daß es gegebenenfalls in Salzform vorliegt.

9. Produkt nach einem der vorhergehenden Ansprüche, **dadurch gekennzeichnet, daß** es sich um folgendes handelt:
3-{4-[3-(2-Fluor-5-trifluormethylphenyl)ureido]phenyl}-1H-pyrrolo[2,3-b]pyridin-2-carboxamid,
3-{4-[3-(2-Fluorphenyl)ureido]phenyl}-1H-pyrrolo[2,3-b]pyridin-2-carboxamid,
3-{4-[3-(2-Methoxyphenyl)ureido]phenyl}-1H-pyrrolo[2,3-b]pyridin-2-carboxamid,
3-{4-[3-(4-Trifluormethylphenyl)ureido]phenyl}-1H-pyrrolo[2,3-b]pyridin-2-carboxamid,
3-{4-[3-(2-Chlor-5-trifluormethylphenyl)ureido]phenyl}-1H-pyrrolo[2,3-b]pyridin-2-carboxamid,
3-{4-[3-(2-Fluor-3-trifluormethylphenyl)ureido]phenyl}-1H-pyrrolo[2,3-b]pyridin-2-carboxamid,
3-{4-[3-(4-Fluor-3-trifluormethylphenyl)ureido]phenyl}-1H-pyrrolo[2,3-b]pyridin-2-carboxamid,
3-{4-[3-(3-Fluor-5-trifluormethylphenyl)ureido]phenyl}-1H-pyrrolo[2,3-b]pyridin-2-carboxamid,
3-{4-[3-(4-Trifluormethoxyphenyl)ureido]phenyl}-1H-pyrrolo[2,3-b]pyridin-2-carboxamid,
3-{4-[3-(3,4-Dimethoxyphenyl)ureido]phenyl}-1H-pyrrolo[2,3-b]pyridin-2-carboxamid,
3-{4-[3-(2,5-Dimethylphenyl)ureido]phenyl}-1H-pyrrolo[2,3-b]pyridin-2-carboxamid,
3-{4-[3-(3-Methoxyphenyl)ureido]phenyl}-1H-pyrrolo[2,3-b]pyridin-2-carboxamid,
3-{4-[3-(3-Trifluormethylphenyl)ureido]phenyl}-1H-pyrrolo[2,3-b]pyridin-2-carboxamid,
3-{4-[3-(3,4-Dimethylphenyl)ureido]phenyl}-1H-pyrrolo[2,3-b]pyridin-2-carboxamid,
3-{4-[3-(2-Methoxy-5-methylphenyl)ureido]phenyl}-1H-pyrrolo[2,3-b]pyridin-2-carboxamid,
3-[4-(3-m-Tolylureido)phenyl]-1H-pyrrolo[2,3-b]pyridin-2-carboxamid,
3-{4-[3-(4-Fluorphenyl)ureido]phenyl}-1H-pyrrolo[2,3-b]pyridin-2-carboxamid,
3-[4-(3-p-Tolylureido)phenyl]-1H-pyrrolo[2,3-b]pyridin-2-carboxamid,
3-{4-[3-(4-Methyl-3-trifluormethylphenyl)ureido]phenyl}-1H-pyrrolo[2,3-b]pyridin-2-carboxamid,
3-{4-[3-(4-Difluormethoxyphenyl)ureido]phenyl}-1H-pyrrolo[2,3-b]pyridin-2-carboxamid,
3-{4-[3-(3,5-Dimethoxyphenyl)ureido]phenyl}-1H-pyrrolo[2,3-b]pyridin-2-carboxamid,
3-{4-[3-(4-Chlor-3-trifluormethylphenyl)ureido]phenyl}-1H-pyrrolo[2,3-b]pyridin-2-carboxamid,
3-{4-[3-(2,5-Dimethoxyphenyl)ureido]phenyl}-1H-pyrrolo[2,3-b]pyridin-2-carboxamid,
3-{4-[3-(3-Fluorphenyl)ureido]phenyl}-1H-pyrrolo[2,3-b]pyridin-2-carboxamid,
3-{4-[3-(2-Methoxy-5-trifluormethylphenyl)ureido]-phenyl}-1H-pyrrolo[2,3-b]pyridin-2-carboxamid,
3-{4-[3-(3-Ethylphenyl)ureido]phenyl}-1H-pyrrolo[2,3-b]pyridin-2-carboxamid.

10. Produkt nach einem der vorhergehenden Ansprüche, **dadurch gekennzeichnet, daß** es sich um folgendes handelt:
3-{4-[3-(2-Fluor-5-trifluormethylphenyl)ureido]phenyl}-7-oxy-1H-pyrrolo[2,3-b]pyridin-2-carboxamid.

11. Produkt nach einem der vorhergehenden Ansprüche, **dadurch gekennzeichnet, daß** es sich um folgendes handelt:
3-{4-[3-(2-Fluor-5-trifluormethylphenyl)ureido]phenyl} 1H-pyrrolo[2,3-c]pyridin-2-carboxamid,
3-{4-[3-(2-Methoxy-5-methylphenyl)ureido]phenyl}-1H-pyrrolo[2,3-c]pyridin-2-carboxamid,
Trifluoracetat von 3-{4-[3-(-Chlorphenyl)ureido]-phenyl}-1H-pyrrolo[2,3-c]pyridin-2-carboxamid,
Trifluoracetat von 3-{4-[3-(3-Chlor-4-fluorphenyl)-ureido]phenyl}-1H-pyrrolo[2,3-c]pyridin-2-carboxamid,
Trifluoracetat von 3-{4-[3-(2-Fluor-5-methylphenyl)-ureido]phenyl}-1H-pyrrolo[2,3-c]pyridin-2-carboxamid,
Trifluoracetat von 3-[4-(3-m-Tolylureido]phenyl}-1H-pyrrolo[2,3-c]pyridin-2-carboxamid,
Trifluoracetat von 3-{4-[3-(2-Acetylamino-5-trifluormethylphenyl)ureido]phenyl}-1H-pyrrolo[2,3-c]pyridin-2-carboxamid,
3-{4-[3-(2-Methoxyphenyl)ureido]phenyl}-1H-pyrrolo[2,3-c]pyridin-2-carboxamid,
3-{4-[3-(2-Trifluormethylphenyl)ureido]phenyl}-1H-pyrrolo[2,3-c]pyridin-2-carboxamid,
3-{4-[3-(3-Trifluormethylphenyl)ureido]phenyl}-1H-pyrrolo[2,3-c]pyridin-2-carboxamid,
3-{4-[3-(4-Fluorphenyl)ureido]phenyl}-1H-pyrrolo[2,3-c]pyridin-2-carboxamid,
3-{4-[3-(4-Trifluormethylphenyl)ureido]phenyl}-1H-pyrrolo[2,3-c]pyridin-2-carboxamid,
3-[4-(3-p-Tolylureido)phenyl]-1H-pyrrolo[2,3-c]pyridin-2-carboxamid,
3-{4-[3-(4-Chlor-3-trifluormethylphenyl)ureido]phenyl}-1H-pyrrolo[2,3-c]pyridin-2-carboxamid,
3-{4-[3-(2-Chlor-5-trifluormethylphenyl)ureido]phenyl}-1H-pyrrolo[2,3-c]pyridin-2-carboxamid,
3-{4-[3-(4-Trifluormethoxyphenyl)ureido]phenyl}-1H-pyrrolo[2,3-c]pyridin-2-carboxamid,
3-{4-[3-(4-Difluormethoxyphenyl)ureido]phenyl}-1H-pyrrolo[2,3-c]pyridin-2-carboxamid,
3-{4-[3-(3,4-Dimethylphenyl)ureido]phenyl}-1H-pyrrolo[2,3-c]pyridin-2-carboxamid,
3-{4-[3-(3,5-Dimethoxyphenyl)ureido]phenyl}-1H-pyrrolo[2,3-c]pyridin-2-carboxamid,
3-{4-[3-(2,5-Dimethylphenyl)ureido]phenyl}-1H-pyrrolo[2,3-c]pyridin-2-carboxamid,
3-{4-[3-(2-Fluorphenyl)ureido]phenyl}-1H-pyrrolo[2,3-c]pyridin-2-carboxamid,
3-{4-[3-(3-Fluorphenyl)ureido]phenyl}-1H-pyrrolo[2,3-c]pyridin-2-carboxamid,
3-{4-[3-(2-Fluor-3-trifluormethylphenyl)ureido]phenyl}-1H-pyrrolo[2,3-c]pyridin-2-carboxamid,
3-{4-[3-(3-Fluor-5-trifluormethylphenyl)ureido]phenyl}-1H-pyrrolo[2,3-c]pyridin-2-carboxamid,
3-{4-[3-(4-Fluor-3-trifluormethylphenyl)ureido]phenyl}-1H-pyrrolo[2,3-c]pyridin-2-carboxamid,
3-{4-[3-(4-Methyl-3-trifluormethylphenyl)ureido]-phenyl}-1H-pyrrolo[2,3-c]pyridin-2-carboxamid,
Trifluoracetat von 3-{4-[3-(3-Methoxyphenyl)ureido] - phenyl}-1H-pyrrolo[2,3-c]pyridin-2-carboxamid,
Trifluoracetat von 3-{4-[3-(3,4-Dimethoxyphenyl)-ureido]phenyl}-1H-pyrrolo[2,3-c]pyridin-2-carboxamid,
Trifluoracetat von 3-{4-[3-(2,5-Dimethoxyphenyl)-ureido]phenyl}-1H-pyrrolo[2,3-c]pyridin-2-carboxamid,
3-[4-(3-o-Tolylureido)phenyl]-1H-pyrrolo[2,3-c]pyridin-2-carboxamid,
3-{4-[3-(4-Methoxyphenyl)ureido]phenyl}-1H-pyrrolo[2,3-c]pyridin-2-carboxamid,
3-{4-[3-(3-Chlor-4-difluormethoxyphenyl)ureido]phenyl}-1H-pyrrolo[2,3-c]pyridin-2-carboxamid,
3-{4-[3-(3,5-Dimethylphenyl)ureido]phenyl}-1H-pyrrolo[2,3-c]pyridin-2-carboxamid,
3-{4-[3-(3-Ethylphenyl)ureido]phenyl}-1H-pyrrolo[2,3-b]pyridin-2-carboxamid.

12. Arzneimittel, **dadurch gekennzeichnet, daß** es ein Produkt der Formel (I) nach einem der Ansprüche 1 bis 11 oder ein Additionssalz dieser Verbindung mit einer pharmazeutisch unbedenklichen Säure oder ein Hydrat oder ein Solvat des Produkts der Formel (I) umfaßt.

13. Pharmazeutische Zusammensetzung, umfassend ein Produkt nach einem der vorhergehenden Ansprüche in Kombination mit einem pharmazeutisch unbedenklichen Träger.

14. Verwendung eines Produkts nach einem der Ansprüche 1 bis 11 als Hemmer einer von einer Kinase katalysierten Reaktion.

15. Verwendung eines Produkts nach Anspruch 14, wobei die Kinase aus der Reihe FAK, KDR, Tie2, Aurora A, Aurora B und CDK2 ausgewählt ist.

16. Verwendung nach Anspruch 15, wobei die Kinase aus der Reihe KDR und Tie2 ausgewählt ist.

17. Verwendung eines Produkts nach einem der Ansprüche 1 bis 11 für die Herstellung eines Arzneimittels zur Behandlung eines pathologischen Zustands.

18. Verwendung nach Anspruch 17, **dadurch gekennzeichnet, daß** es sich bei dem pathologischen Zustand um Krebs handelt.
